# EUROPEAN PATENT APPLICATION

(11) **EP 2 019 100 A1**
(43) Date of publication of application: **28.01.2009**
(21) Application number: 07014219.5
(22) Date of filing: 19.07.2007
(51) Int. Cl.: C07D 401/12, C07D 401/14, A61K 31/435, A61P 3/04, A61P 3/10, A61P 15/10

(54) **Substituted heteroarylpiperidine derivatives as melanocortin-4 receptor modulators**

(71) Applicant: Santhera Pharmaceuticals (Schweiz) AG, 4410 Liestal (CH)
(72) Inventor: Henneböhle, Marco, 79618 Rheinfelden (DE); Herzner, Holger, 79595 Rümmingen (DE); Soeberdt, Michael, 79618 Rheinfelden (DE); Weyermann, Philipp, 4450 Sissach (CH); Nordhoff, Sonja, 4144 Arlesheim (CH); Feurer, Achim, 79424 Auggen (DE)
(74) Representative: Grünecker, Kinkeldey, Stockmair & Schwanhäusser Anwaltssozietät

(57) **Abstract**

The present invention relates to substituted heteroarylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

## Description

### Field of the Invention

The present invention relates to substituted heteroarylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified and these are expressed in different tissues.

MC-1 R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in humans.
The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1 R. The mechanism by which agonism of MC-1R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti (A^{vy}) mouse which ectopically expresses an antagonist of the MC-1R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, (1998)). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (M.J. Owen and C.B. Nemeroff, "Physiology and pharmacology of corticotrophin releasing factor." Pharmacol. Rev. 43: 425-473 (1991)). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Shigeyuki Chaki et al, "Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor", J. Pharm. Exp. Ther. 304(2), 818-826 (2003)).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (D.L. Marks et al. "Role of the central melanocortin system in cachexia." Cancer Res. 61: 1432-1438 (2001)).

Modulators of the melanocortin receptor are already known from the literature. WO 2004/024720 A1 describes piperazine urea derivatives which are selective agonists of the human melanocortin-4 receptor and as such they are claimed to be useful in the treatment of prevention of obesity-related disorders.

WO 2005/047253 A1 describes 4,4-disubstituted piperidine derivatives which are postulated to function as melanocortin receptor agonists.

Substituted piperidine derivatives are also described in DE 103 00973 which relates to carboxylic acids and esters having a piperidine ring or a piperazine ring as the central core of the molecule and wherein the core is further substituted in the para-position by a 5-7-membered heterocycle, a phenyl ring, a pyridine ring or a thiazole ring. Said rings are optionally substituted by an ester group. The compounds are used in the preparation of a medicament for the treatment of headaches, non-insulin dependent diabetes mellitus (NIDDM), cardiovascularic diseases, morphintolerance, diseases of the skin, inflammations, allergic rhinitis, asthma, diseases with vascular dilatation and, consequently, with reduced blood circulation in tissues, acute or preemptive treatment of menopausal hot flashes of women with an estrogen deficiency or for the treatment of pain.

In view of the unresolved deficiencies in treatment of various diseases and disorders as discussed above, it is an object of the present invention to provide novel substituted piperidine derivatives with improved ability to cross the blood brain barrier, which are useful as melanocortin-4 receptor modulators to treat cancer cachexia, muscle wasting, anorexia, anxiety, depression, obesity, diabetes, sexual dysfunction and other diseases with MC-4R involvement.

### Summary of the Invention

The present invention relates to substituted heteroarylpiperidine derivatives of structural formula (I) 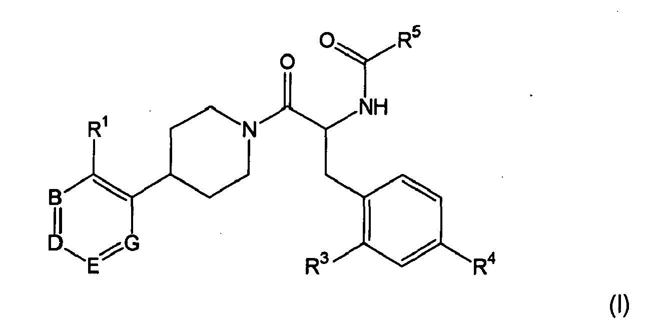 wherein R¹, R³, R⁴, R⁵, B, D, E and G are defined as described below. The heteroarylpiperidine derivatives of structural formula (I) are effective as melanocortin receptor modutators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to substituted heteroarylpiperidine derivatives useful as melanocortin receptor modulators, in particular, selective MC-4R agonists and MC-4R antagonists.

The compounds of the present invention are represented by structural formula (I) 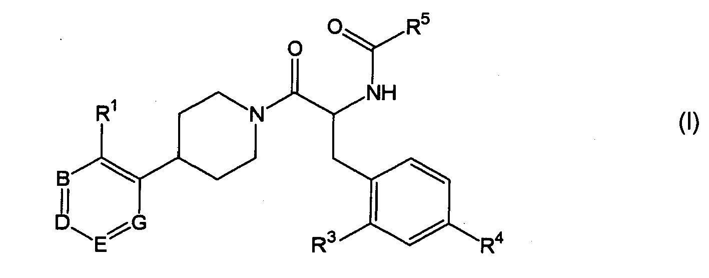 and the enantiomers, diastereomers, tautomers, solvates and pharmaceutically acceptable salts thereof,
wherein
- R¹ is: -N(R¹⁰)-(C(R⁶)₂)ₘ-T
-(C(R⁶)₂)ₗ-T, or
-O-(C(R⁶)₂)ₘ-T;
- R⁶ is: independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
- T is: NR⁷R⁸,
morpholine, 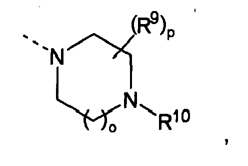 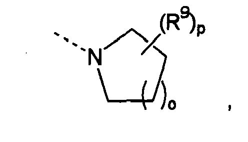 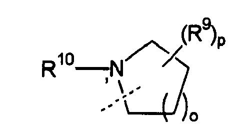 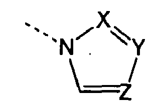 or 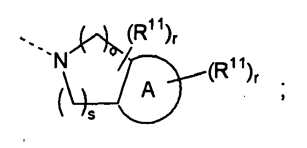
- R⁷ and R⁸ are: independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
- R⁹ is: independently selected from
halogen,
CN,
OH,
C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH;
- R¹⁰ is: H, or
C₁-C₆-alkyl;
- R¹¹ is: independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
- X is: CH or N;
- Y is: CH or N;
- Z is: CH or N;
- A is: a 3-7-membered saturated, unsaturated or aromatic ring containing 0-2 nitrogen atoms;
- B is: CR² or N;
- G is: CR² or N;
- D is: CR² or N;
- E is: CR² or N;
with the proviso that one or two of the variables B, G, D and E must be N;
- R² is: independently selected from
H,
F,
Cl,
CH₃,
OCH₃, and
CF₃;
- R³ is: H,
Cl,
F, or
CH₃;
- R⁴ is: Cl or F;
- R⁵ is: 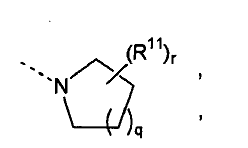 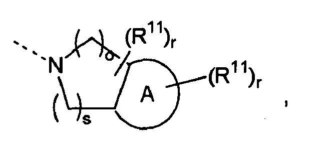 morpholine, optionally substituted by 1 to 3, same or different substituents R¹⁴, or
NR¹²R¹³_{;}
- R¹² and R¹³ are: independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylne-N-(C₁₋₆-alkyl)₂;
- R¹⁴ is: C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂;
- I is: 0, 1, 2, 3, or 4;
- m is: 0, 1, 2, 3, or 4;
- o is: 0, 1, or 2;
- p is: 0, 1, 2, 3, or 4;
- q is: 0, 1, 2, or 3;
- r is: 0, 1, 2, 3, or 4 and
- s is: 1, or 2.

Preferably, the compounds according to formula (I) adopt the structural conformation of the following stereoisomer formula (I'): 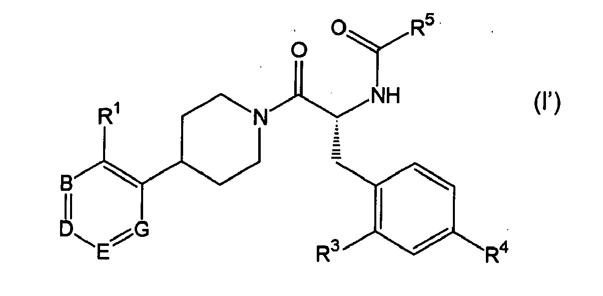 wherein B, G, D, E, R¹, R³, R⁴ and R⁵ are as defined above.

The moiety 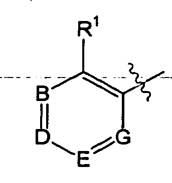 in general formula (I) is selected from the following structures: 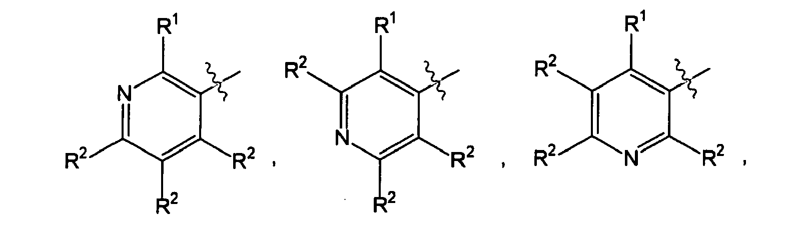 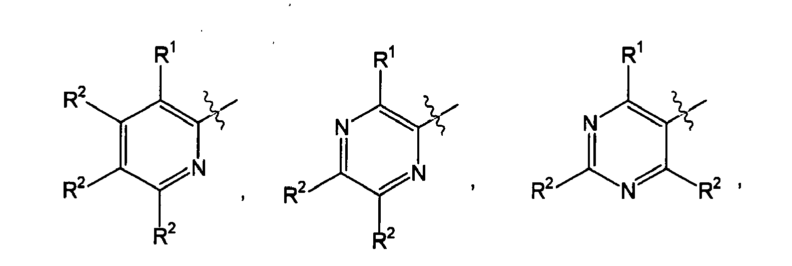 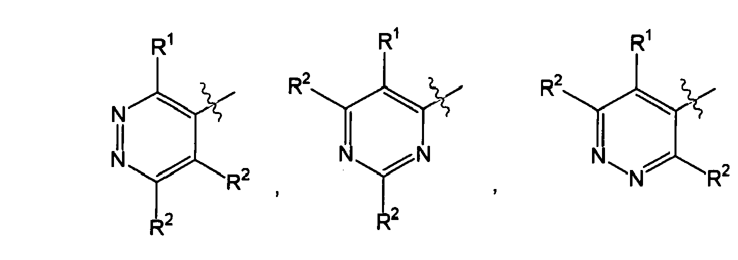 and

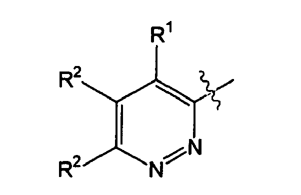

Therein, the variant R² is defined as above. In a preferred embodiment of the present invention, R² represents H, Cl, F or CH₃. More preferred, R² represents H or CH₃.

Preferred embodiments of the moiety 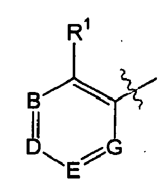 are the following structures:

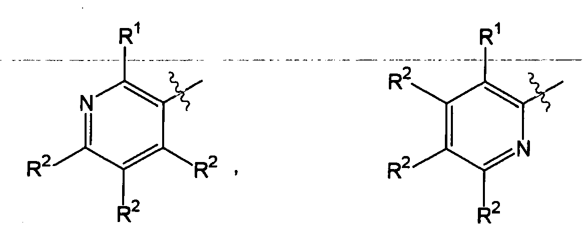

In a preferred embodiment of the present invention, the variant R¹ represents -N(R¹⁰)-(C(R⁶)₂)ₘ-T, -(C(R⁶)₂)ₗ-T, or -O-(C(R⁶)₂)ₘ-T wherein R⁶ and R¹⁰ are preferably independently selected from the group consisting of H and C₁₋₆-alkyl.

It is further preferred that R³ represents H, Cl, or CH₃, more preferably Cl. In an alternative embodiment, R³ preferably represents F.

Preferably, R⁴ represents Cl.

In a preferred embodiment of the present invention, the variant R⁵ represents 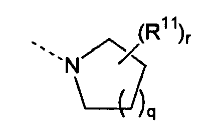 wherein r preferably assumes the number 0 or 1 and q preferably assumes the number 1 or 2.

In a further preferred embodiment at least one of R⁷ and R⁸ is selected from the group consisting of C₁₋₆-alkyl, C₂₋₆-alkenyl, C₂₋₆-alkinyl and C₂₋₆-alkylene-O-C₁₋₆-alkyl, more preferably from C₂₋₆-alkenyl, C₂₋₆-alkinyl and C₂₋₆-alkylene-O-C₁₋₆-alkyl.

It is preferred that R⁹ is independently selected from the group consisting of halogen, CN, OH, C₁₋₆-alkyl optionally substituted with 1 to 3 substituents selected from halogen, CN and OH, and O-C₁₋₆-alkyl optionally substituted with 1 to 3 substituents selected from halogen, CN and OH.

The variant I is preferably selected from 2 or 3.

The variant m is preferably selected from 2, 3 or 4, more preferably from 2 or 3.
As regards compounds of formula (I), T is preferably selected from the group consisting of the following radicals:

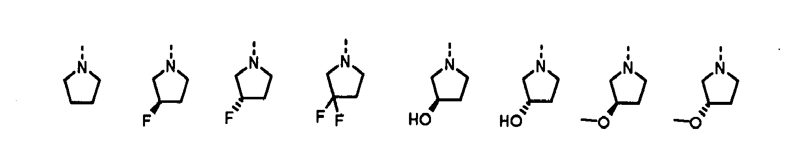

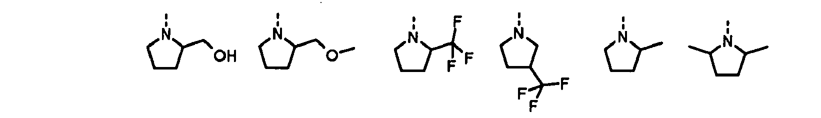

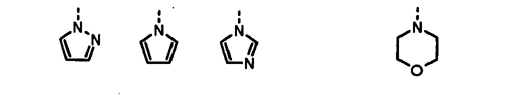

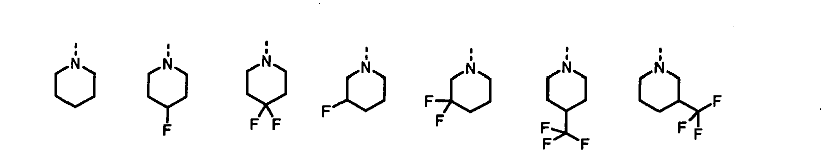

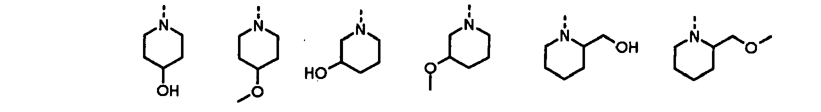

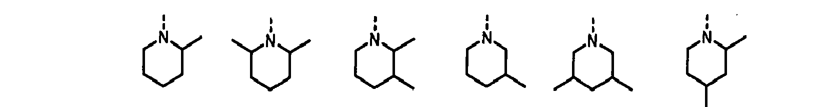

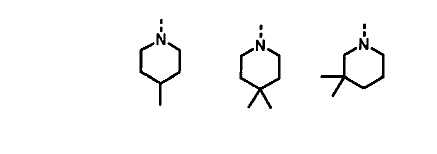

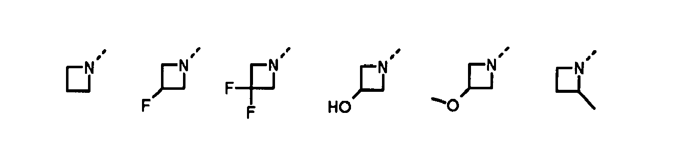

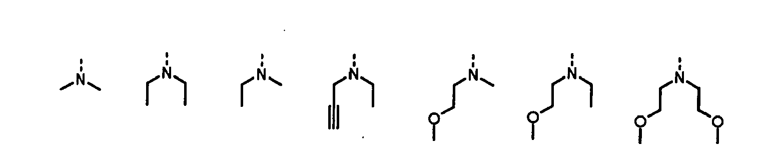

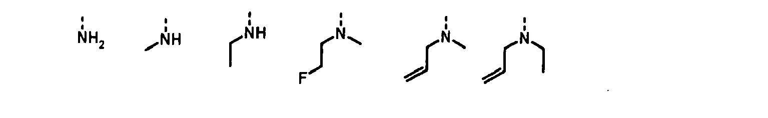

In a further preferred embodiment, R⁵ is preferably selected from the group consisting of

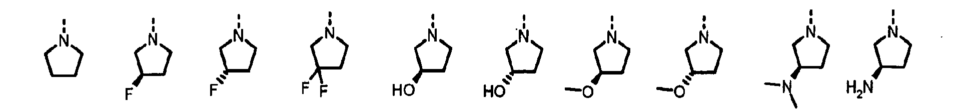

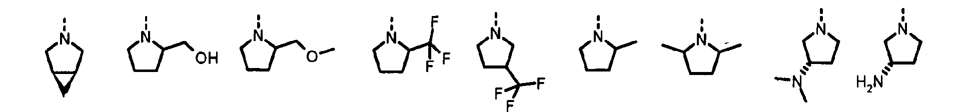

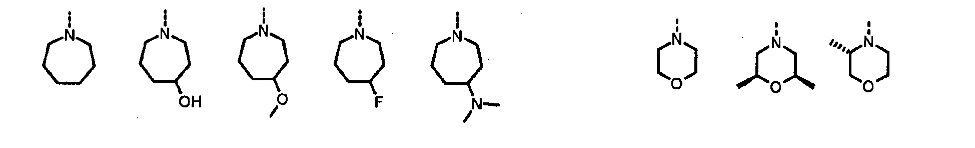

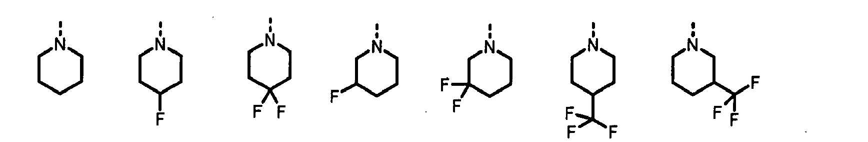

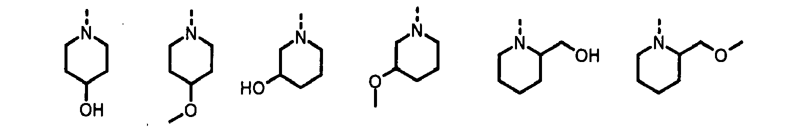

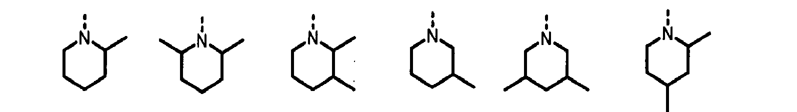

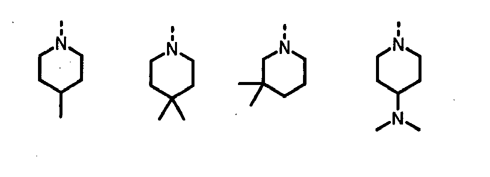

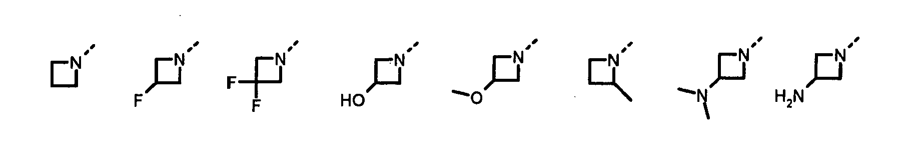

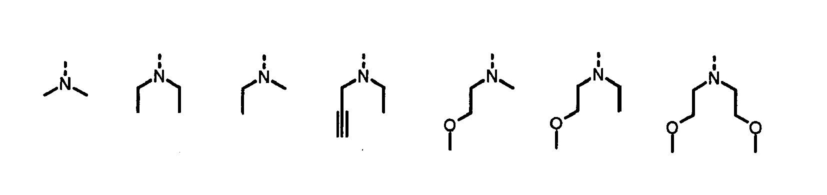

Compounds of the formula (I) in which some or all of the above-mentioned groups have the preferred or more preferred meanings are also an object of the present invention.

In the above and the following, the employed terms have the meaning as described below:

Alkyl is a straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 2 to 6 carbon atoms and which contains at least one carbon-carbon double bond, such as vinyl, allyl, 1-propenyl, 2-butenyl, 2-methyl-2-butenyl, isopropenyl, pentenyl, or hexenyl.

Alkinyl is a straight chain or branched alkyl having 2 to 6 carbon atoms and which contains at least one carbon-carbon triple bond, such as ethinyl, 1-propinyl, 1-butinyl, 2-butinyl, pentinyl or hexinyl.

A 3-7-membered, saturated, unsaturated or aromatic ring containing 0-2 nitrogen atoms encompasses a 3-7-membered saturated carbocycle such as cyclopropyl, cyclobutyl, cyclopentyl, cyclohexyl or cycloheptyl. Said term further encompasses 3-7-membered unsaturated carbocycles such as cyclobutenyl, cyclopentenyl, cyclohexenyl, cycloheptenyl, cyclohexa-1,4-diene or cycloheptadienes, or aromatic rings such as benzene. Nitrogen-containing, 3-7-membered, saturated, unsaturated or aromatic heterocycles are further encompassed by the above term. Examples thereof include azetidine, pyrrolidine, piperidine, azepane, piperazine, pyridine, pyrimidine, pyrazine, pyrrole, imidazole, and pyrazole.

The compounds of structural formula (I) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

The compounds of structural formula (I) are particularly useful as antagonists of MC-4R. Thus, they are preferably used for the preparation of a medicament for the treatment and/or prevention of cancer cachexia, muscle wasting, anorexia, anxiety and depression.

### Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

The compounds of formula (I) are melanocortin receptor modulators and as such are useful in the treatment, control or prevention of diseases, disorders or conditions responsive to the inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance) and male and female sexual dysfunction (including impotence, loss of libido and erectile dysfunction).

The compounds of formulas (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formula (I) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formula (I), the terms "A moiety", "B moiety" and "C moiety" are used below. This moiety concept is illustrated below:

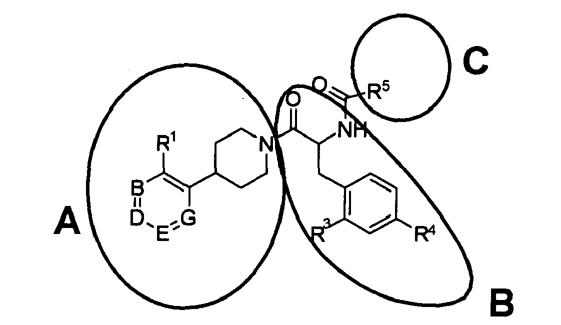

The preparation of the compounds of the present invention may be carried out via sequential or convergent synthetic routes. The skilled artisan will recognize that, in general, the A and B moieties of a compound of formula (I) are connected via amide bonds. The skilled artist can, therefore, readily envision numerous routes and methods of connecting the two moieties via standard peptide coupling reaction conditions.

The phrase "standard peptide coupling reaction conditions" means coupling a carboxylic acid with an amine using an acid activating agent such as EDCI, dicyclohexylcarbodiimide and benzotriazol-1-yloxytris(dimethylamino)-phosphonium hexafluorophosphate, in a inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The B and C moieties of a compound of formula (I) are linked together via a urea function. The skilled artist can, therefore, readily envision numerous routes and methods of connecting the two moieties using different well known methods.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following schemes and examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings:
- Ac₂O: acetic anhydride
- Boc: tert-butoxycarbonyl
- Bu: butyl
- BuLi: butyllithium
- DCM: dichloromethane
- DEAD: diethyl azodicarboxylate
- DIAD: diisopropyl azodicarboxylate
- DIEA: ethyl-diisopropylamine
- DMA: N, N-dimethylacetamide
- DMAP: 4-dimethylaminopyridine
- DMF: N,N-dimethylformamide
- DMS: dimethylsulfide
- DMSO: dimethylsulfoxide
- dppf: 1,1'-bis(diphenylphosphino)-ferrocen
- EDCI: 1-(3-dimethylaminopropyl)-3-ethylcarbodiimide hydrochloride
- Et₂O: diethyl ether
- EtOAc: ethyl acetate
- EtOH: ethanol
- h: hour(s)
- HOBt: 1-hydroxybenzotriazole hydrate
- HTMP: 2,2,6,6-tetramethylpiperidine
- MeCN: acetonitrile
- MeOH: methanol
- NMM: N-methylmorpholine
- PG: protecting group
- PPh₃: triphenylphosphine
- TEA: triethylamine
- TFA: trifluoroacetic acid
- THF: tetrahydrofurane
2-Fluoro-3-iodo-pyrazine was prepared as described in Tetrahedron 1998, 54, 4899-4912. 4-Fluoro-3-iodo-pyridine was prepared as described in Tetrahedron 1993, 49, 49-64.

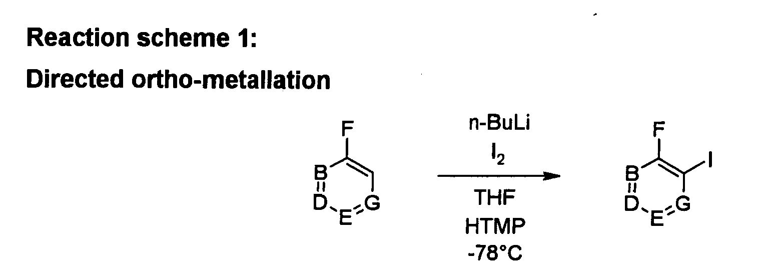

The starting material for the synthesis of heteroarylpiperidines, ortho-fluoro-iodopyridines, -pyridazines and -pyrazines, can be prepared as shown in Reaction scheme 1. A fluoro-substituted heteroaryl can be metallated with an amide prepared from a reagent such as n-butyllithium and an amine such as diisopropylamine or 2,2,6,6-tetramethylpiperidine at an appropriate temperature in a suitable solvent such as THF. The resulting lithio derivatives can subsequently be reacted with iodine to yield the desired compounds.

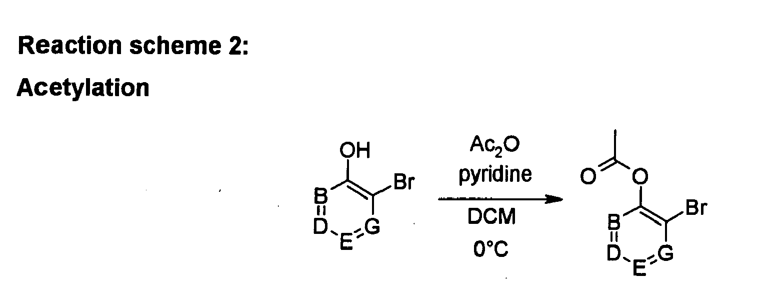

Another starting material for the synthesis of heteroarylpiperidines, ortho-acetoxy-bromopyridines, -pyridazines and -pyrazines, can be prepared as shown in Reaction scheme 2. A bromoheteroaryl containing a hydroxy group in ortho-position to the bromo atom can be reacted with an acetylating reagent such as acetic anhydride in the presence of a suitable base such as pyridine in an appropriate solvent like DCM at a suitable temperature.

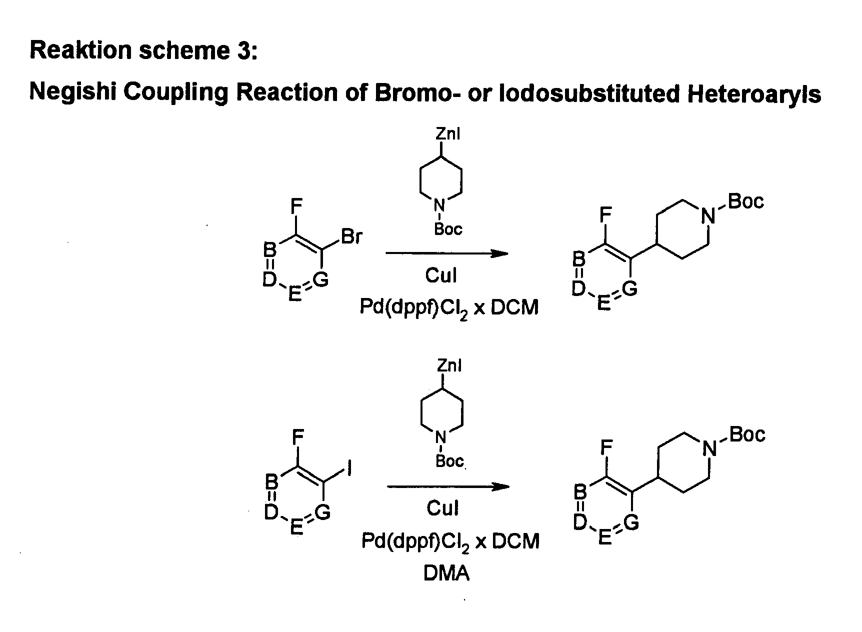

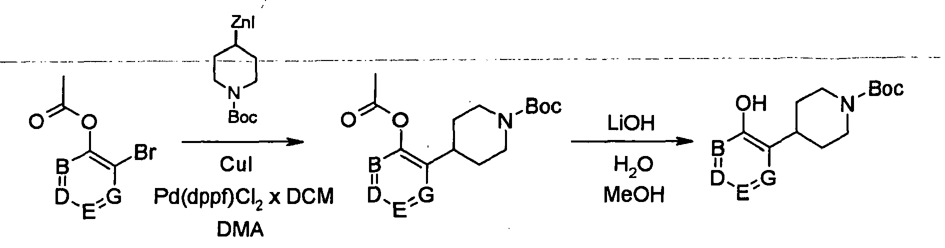

As shown in Reaction scheme 3, ortho-fluoro-bromopyridines, pyridazines and -pyrazines can be subjected to a Negishi coupling with (1-tert-butoxycarbonylpiperidin-4-yl)(iodo)zinc (J. Org. Chem. 2004, 69, 5120-5123) in the presence of copper(I) iodide and dichloro(1,1'-bis(diphenyl-phosphino)-ferrocene)palladium(II) DCM adduct in an inert solvent such as DMA to yield the resulting arylpiperidine. The same product is obtained, when ortho-fluoro-iodoopyridines, -pyridazines and -pyrazines are used as starting material.
The Negishi coupling can alternatively be performed using the ortho-acetoxysubstituted bromopyridines, -pyridazines and -pyrazines from Reaction scheme 2 as starting material. The free alcohol is obtained by saponification of the acetic acid ester with a base such as lithium hydroxide in a suitable solvent such as mixture of water and methanol.

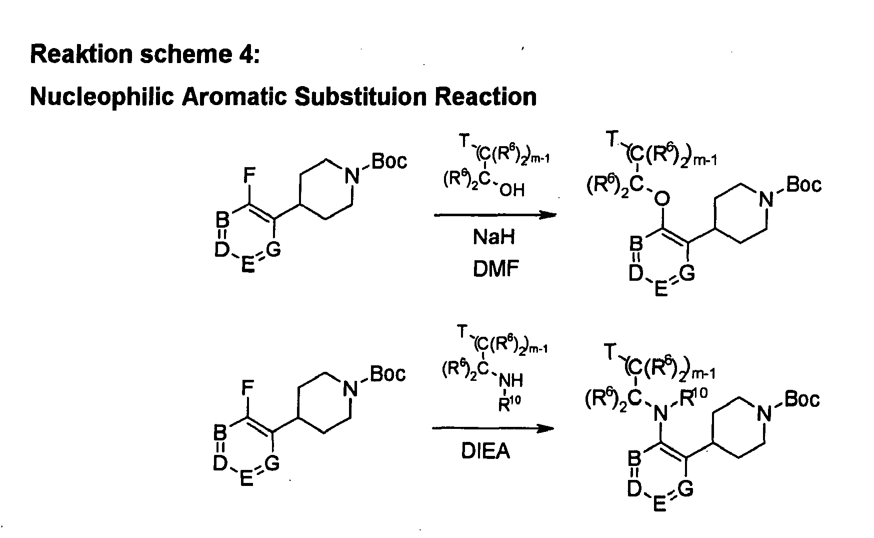

As shown in Reaction scheme 4, fluoro-substituted pyridyl-, pyridazyl- and pyrazinylpiperidines can be subjected to a nucleophilic aromatic substitution reaction with a ω-T-capped alkylalcohol in the presence of a base such as sodium hydride in a solvent such as DMF at a suitable temperature to obtain the Boc-protected A moiety.
Alternatively, the fluoro-substituted heteroarylpiperidines can also be reacted with a ω-T-capped primary or secondary alkylamine in the presence of a base like BuLi or DIEA in an appropriate solvent like THF or without a solvent at a suitable temperature to obtain the Boc-protected A moiety.

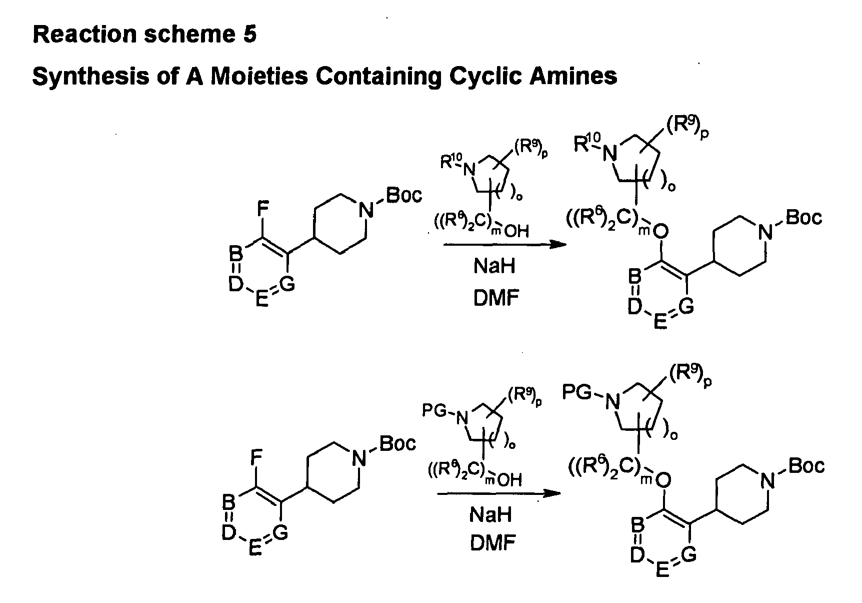

As shown in Reaction scheme 5, the intermediate product from Reaction scheme 3, optionally substituted fluoropyridines, -pyridazines and -pyrazines, can also be subjected to a nucleophilic aromatic substitution reaction with an alcohol which contains a cyclic tertiary amine moiety, in the presence of a base such as sodium hydride in a suitable solvent such as DMF to give the Boc-protected A moieties.

Similarly, an alcohol containing a protected cyclic secondary amine moiety can be introduced as building block using the conditions described above. The protecting group has to be orthogonal to the Boc-protecting group used for protection of the piperidine. After coupling of the A moiety with the B-C moiety this protecting group can oe removed using standard methods.

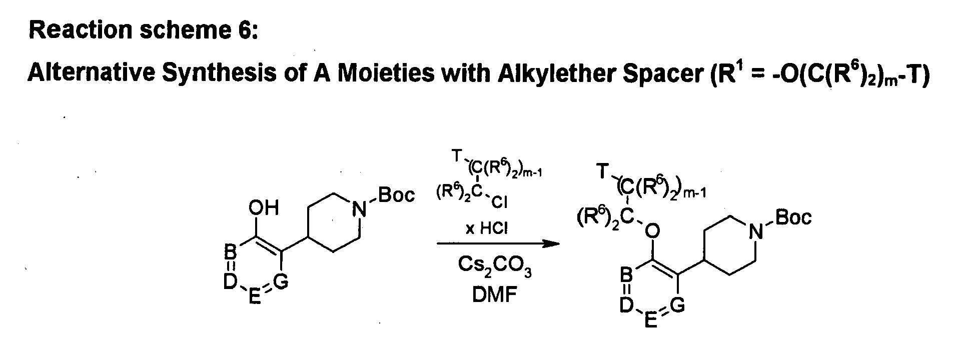

The synthesis of A Moieties bearing an alkylether spacer (R¹ = -O(C(R⁶)₂)ₘ-T) can alternatively be performed as depicted in Reaction scheme 6. The Boc-protected piperidine is reacted with an alkylchloride or alkylbromide bearing the capping group T in the presence of a base such as Cs₂CO₃ or NaH in an appropriate solvent such as DMF to give the Boc-protected A moiety.

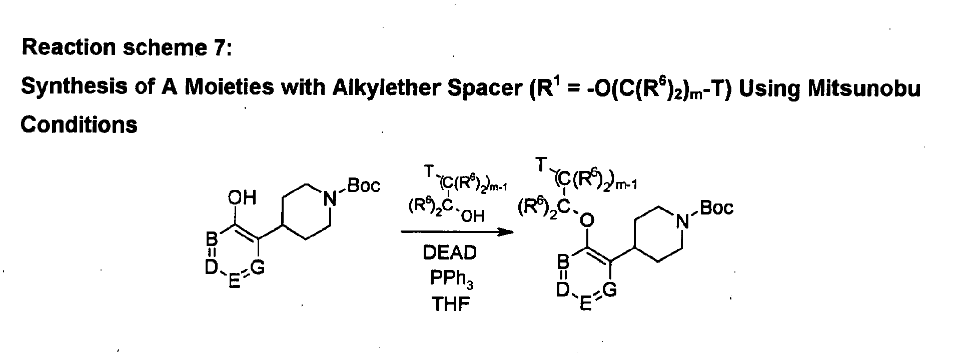

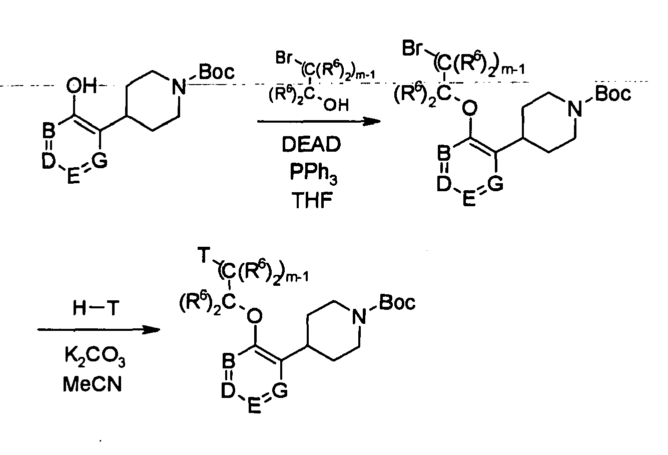

As shown in Reaction scheme 7, the intermediate product from Reaction scheme 3, optionally substituted (hydroxyheteraoaryl)piperidines, can also be alkylated with an ω-T-capped alkylalcohol in the presence of a reagent such as DEAD or DIAD and a phosphine such as PPh₃ in a suitable solvent such as THF to give the Boc-protected A moieties.

Similarly, the same intermediate can be reacted with an ω-bromo alkylalcohol, using the reaction conditions described above, to give access to the corresponding ether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

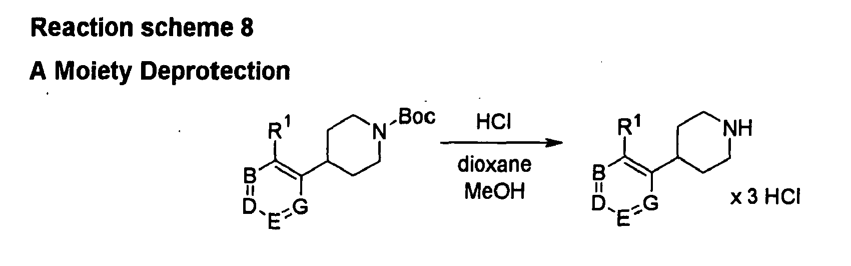

Generally, the starting material of Boc-protected heteroarylpiperidine (A moiety) can be deprotected in the presence of TFA/CH₂Cl₂, HCl/EtOAc, HCl/dioxane or HCl in MeOH/dioxane with or without a cation scavenger, such as dimethyl sulfide (DMS) before being subjected to the coupling procedure. It can be converted to the free base before being subjected to the coupling procedure or in some cases used as the salt.

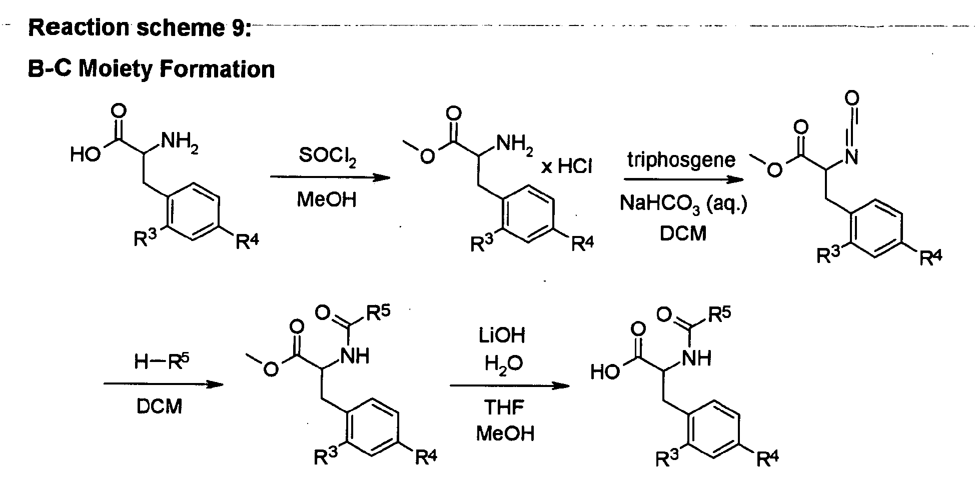

The B-C moieties can be synthesized as shown in Reaction scheme 9. Optionally substituted phenylalanine can be converted to the corresponding methyl ester hydrochloride using an activating reagent such as thionyl chloride or oxalyl chloride in methanol. Amino acid methyl ester hydrochloride can be reacted with a reagent such as triphosgene in the presence of a base such as NaHCO₃ (aq.) in a suitable solvent such as DCM to yield the isocyanate which can subsequently be reacted with an amine R⁵-H in a suitable solvent such as DCM. The ester function can be hydrolyzed with a base such as LiOH in a suitable solvent or solvent mixture such as water/THF/methanol to give access to the B-C-moiety.

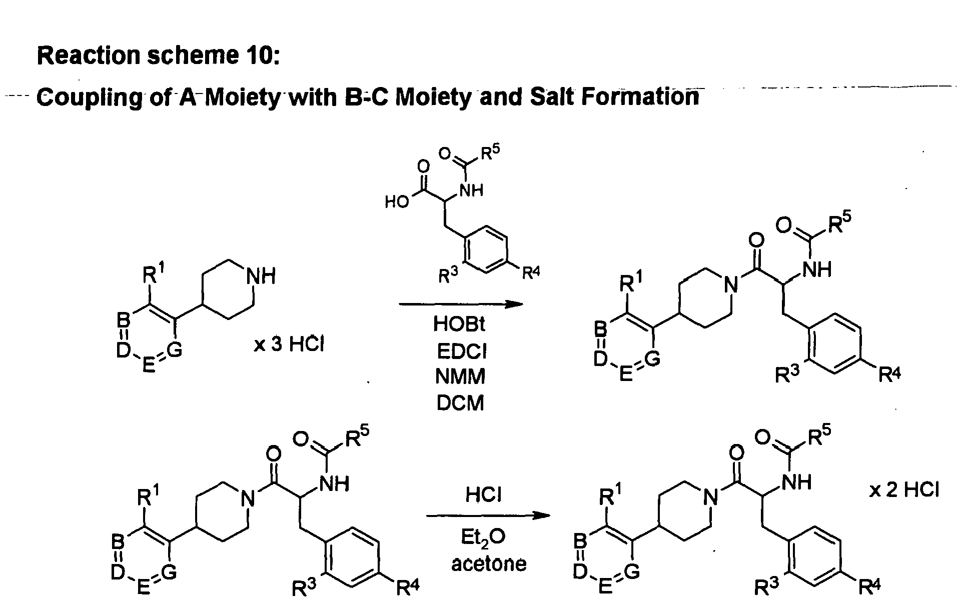

As shown in Reaction scheme 10, A moieties can be coupled with B-C moieties in the presence of EDCI/HOBt, a base such as N-methylmorpholine (NMM) and a solvent such as dichloromethane (DCM). A suitable solvent, such as DCM, DMF, THF or a mixture of the above solvents, can be used for the coupling procedure. A suitable base includes triethylamine (TEA), diisopropylethylamine (DIEA), N-methylmorpholine (NMM), collidine or 2,6-lutidine. A base may not be needed when EDCI/HOBt is used.

Generally after the reaction is completed, the reaction mixture can be diluted with an appropriate organic solvent, such as EtOAc, DCM or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.
The product can be transferred to a pharmaceutically acceptable salt such as a hydrochloride, using HCl in a solvent or solvent mixture such as diethyl ether/acetone.

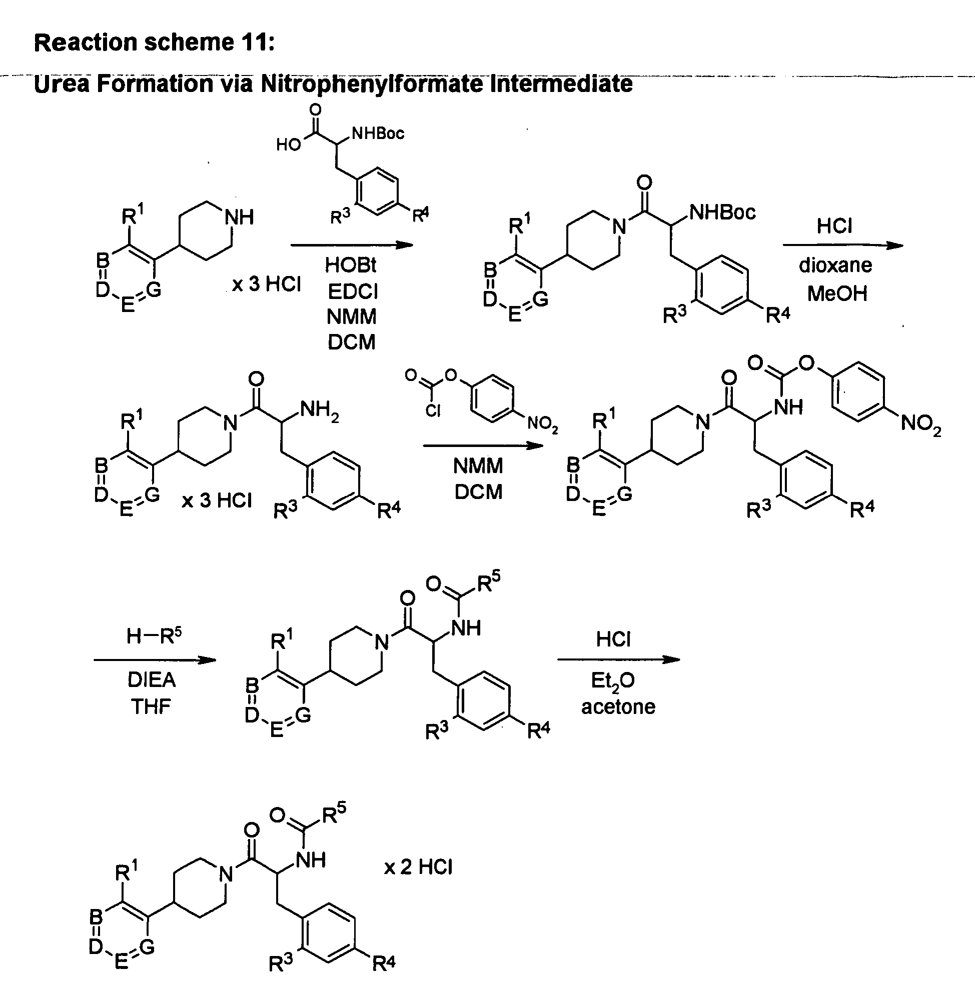

The three moieties can also be combined stepwise, as shown in Reaction scheme 11. An appropriate A moiety is coupled to a Boc-protected B moiety in the presence of EDCl/HOBt, a base such as N-methylmorpholine (NMM) and a solvent such as dichloromethane (DCM) followed by Boc deprotection with the aid of hydrogen chloride in a mixture of dioxane and methanol. The product can be reacted with 4-nitrophenyl chloroformate in the presence of a base such as NMM in an appropriate solvent such as DCM to yield the 4-nitrophenyl carbamate which subsequently can be treated with an amine H-R⁵ in the presence of a base such as DIEA in an appropriate solvent such as THF to give access to the target compound. The final product can be converted to a pharmaceutically acceptable salt as described above.

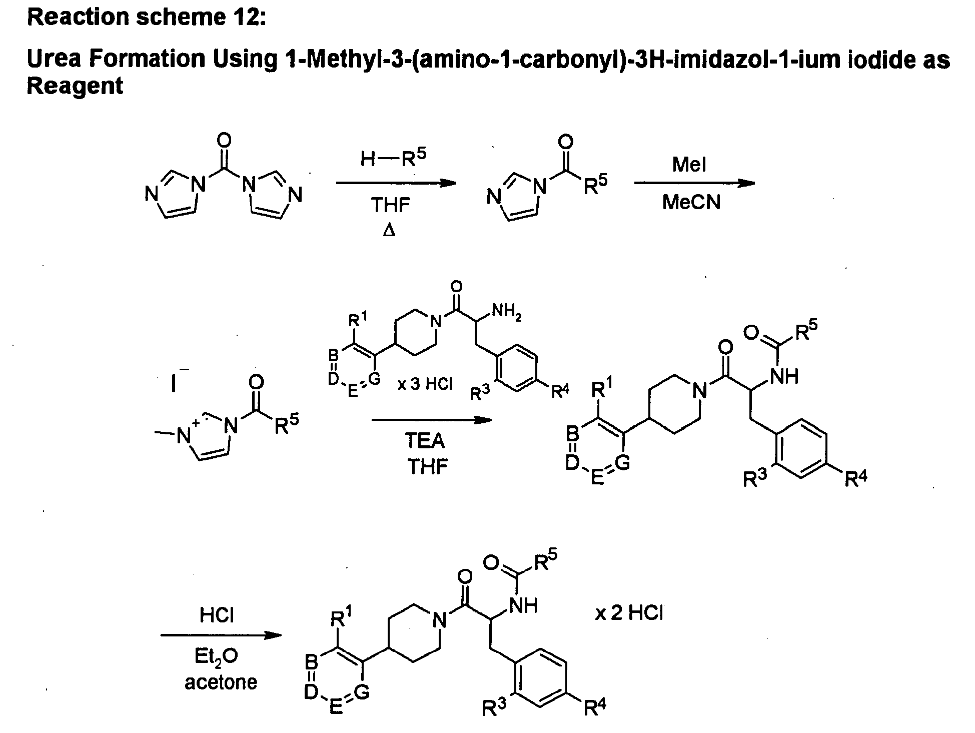

As shown in Reaction scheme 12 1,1'-carbonyldiimidazole can be reacted with an amine in an appropriate solvent such as THF at a suitable temperature. The product of this reaction is further reacted with methyl iodide in a suitable solvent such as acetonitrile to yield the 1-methyl-3-(amino-1-carbonyl)-3H-imidazol-1-ium iodide. This activated species is reacted with a deprotected A-B moiety in the presence of a base such as triethylamine in a suitable solvent such as THF to yield the final product. The final product can be converted to a pharmaceutically acceptable salt as described above.

### Analytical LC-MS

The compounds of the present invention according to formula (I) were analyzed via analytical LC-MS. The conditions used in the analysis are summarized below.

### Analytical conditions summary:

LC10Advp-Pump (Shimadzu) with SPD-M10Avp UV/Vis diode array detector and QP2010 MS-detector in ESI+ modus with UV-detection at 214, 254 and 275 nm,
Column: Waters XTerra MS C18, 3.5 µm, 2.1 * 100 mm,
linear gradient with acetonitrile in water (0.1 % HCOOH)
Flow rate of 0,4 ml/min;
Mobile Phase A: water (0.1% HCOOH)
Mobile Phase B: acetonitrile (0.1 % HCOOH)

### Gradient A:

| start | concentration | 1 % acetonitrile |
|---|---|---|
| 9.00 | B.Conc | 30 |
| 10.00 | B.Curve | 3 |
| 12.00 | B.Conc | 99 |
| 15.00 | B.Conc | 99 |
| 15.20 | B.Conc | 1 |
| 18.00 | Pump STOP | |

### Gradient B:

| start | concentration | 10% acetonitrile |
|---|---|---|
| 10.00 | B.Conc | 60 |
| 11.00 | B.Curve | 2 |
| 12.00 | B.Conc | 99 |
| 15.00 | B.Conc | 99 |
| 15.20 | B.Conc | 10 |
| 18.00 | Pump STOP | |

The following tables describe detailed examples of the invention which can be prepared according to the Reaction schemes 1 to 12. These examples are, however, not construed to limit the scope of the invention in any manner.

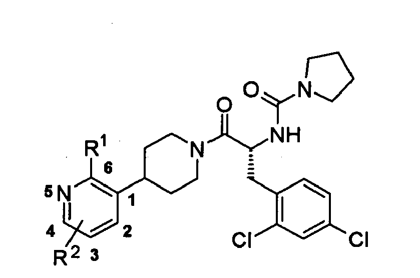

**Table 1:**

| | | | | HPLC | | MS | |
|---|---|---|---|---|---|---|---|
| No. | salt | R¹ | R² | t_{R} (min) | method | MW (calc.) free base | [M+H]⁺ (found) |
| 1 | 2 x HCl | 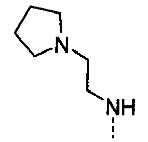 | 4-Me | 4.36 | B | 600 | 601 |
| 2 | 2 x HCl | 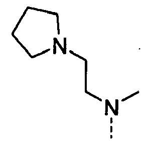 | 4-Me | 5.34 | B | 614 | 615 |
| 3 | 2x HCOOH | 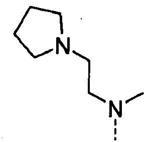 | 3-Me | 3.99 | B | 614 | 615 |
| 4 | 2 x HCl | 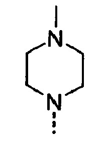 | 4-Me | 5.60 | B | 586 | 587 |
| 5 | 2x HCOOH | 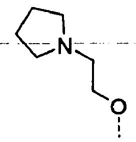 | H | 5.80 | B | 587 | 590 |
| 6 | 2 x HCl | 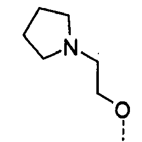 | 4-Me | 6.48 | B | 601 | 604 |
| 7 | 2x HCOOH | 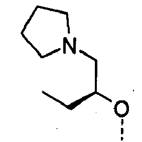 | H | 6.32 | B | 615 | 616 |
| 8 | 2x HCOOH | 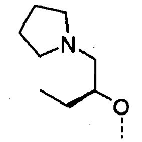 | 4-Me | 6.74 | B | 629 | 630 |
| 9 | 2x HCOOH | 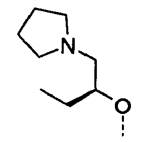 | 3-Me | 6.90 | B | 629 | 630 |

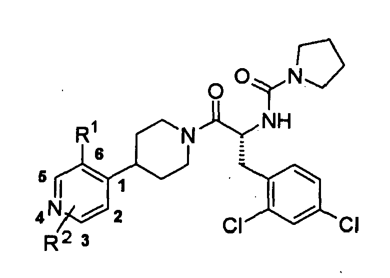

**Table 2:**

| | | | | HPLC | | MS | |
|---|---|---|---|---|---|---|---|
| No. | salt | R¹ | R² | t_{R} (min) | method | MW (calc.) free base | [M+H]⁺ (found) |
| 10 | 2 x HCl | 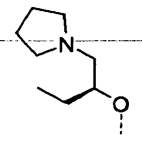 | H | 4.04 | B | 615 | 618 |

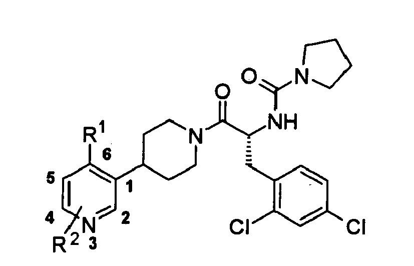

**Table 3:**

| | | | | HPLC | | MS | |
|---|---|---|---|---|---|---|---|
| No. | salt | R¹ | R² | t_{R} (min) | method | MW (calc.) free base | [M+H]⁺ (found) |
| 11 | 2 x HCl | 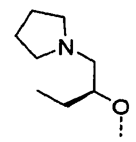 | H | 3.64 | B | 615 | 618 |

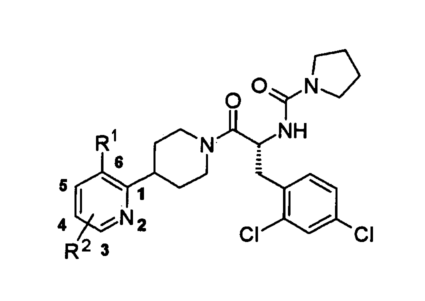

**Table 4:**

| | | | | HPLC | | MS | |
|---|---|---|---|---|---|---|---|
| No. | salt | R¹ | R² | t_{R} (min) | method | MW (calc.) free base | [M+H]⁺ (found) |
| 12 | 2 x HCl | 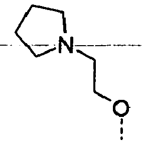 | H | 7.48 | A | 587 | 588 |
| 13 | 2 x HCl | 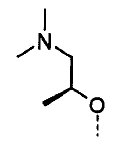 | H | 4.18 | B | 575 | 576 |
| 14 | 2 x HCl | 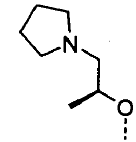 | H | 4.47 | B | 601 | 602 |
| 15 | 2 x HCl | 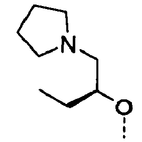 | H | 4.73 | B | 615 | 618 |
| 16 | - | 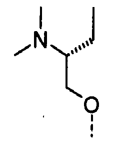 | H | 8.99 | A | 589 | 590 |
| 17 | 2 x HCl | 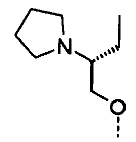 | H | 4.66 | B | 615 | 617 |
| 18 | - | 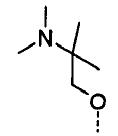 | H | 5.15 | A | 589 | 590 |
| 19 | 2 x HCl | 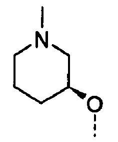 | H | 4.28 | B | 587 | 588 |

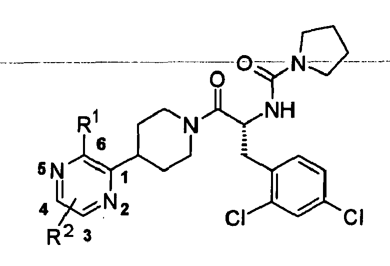

**Table 5:**

| | | | | HPLC | | MS | |
|---|---|---|---|---|---|---|---|
| No. | salt | R¹ | R² | t_{R} (min) | method | MW (calc.) free base | [M+H]⁺ (found) |
| 20 | 2 x HCl | 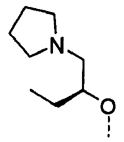 | H | 6.11 | B | 616 | 621 |

The following examples are provided to illustrate the invention and are not limiting the scope of the invention in any manner.

### Common Intermediates:

### 1-tert-Butoxycarbonylpiperidin-4-yl)(iodo)zinc:

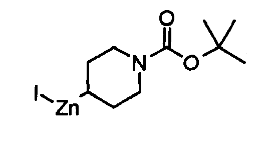

**Zinc activation.** A Schlenk flask was charged with Celite (174 mg) and dried by heating *in vacuo*. Then zinc dust (883 mg) and dry *N,N*-dimethylacetamide (2 ml) were added. The mixture was stirred at room temperature while a 7:5 v/v mixture of chlorotrimethylsilane (153 µl) and 1,2-dibromoethane (108 µl) as solution in *N,N*-dimethylacetamide (1 ml) was added at a rate to maintain the temperature below 65 °C (∼5 min). The resulting slurry was aged for 15 min.

**Zink insertion.** A solution of Boc-4-iodopiperidine (3.36 g) in *N,N*-dimethylacetamide (6 ml) was slowly added to the mixture described above at a rate to maintain a temperature below 65 °C (∼5 min). The resulting reaction mixture was then aged at room temperature for 30 min. The suspension was filtered through a frit under argon to remove all solids. The resulting pale yellow solution was stored at room temperature under argon and was directly used for the coupling reactions.

### (S)-1-Pyrrolidin-1-yl-butan-2-ol:

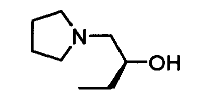

Pyrrolidine (5.76 ml) was added to a mixture of (S)-(-)-1,2-epoxybutane (5.00 ml) and water (25 ml). The reaction mixture was vigorously stirred overnight at room temperature. More water (25 ml) was added, and the organic materials were extracted with ether (2 x 100 ml). The combined organic layer was dried (Na₂SO₄), filtered and concentrated in *vacuo*. In order to get rid of volatile impurities several co-evaporations with ether and toluene were carried out (each with 2 x 10 ml) to furnish the desired compound in form of a colorless liquid.

### 2-Dimethylamino-2-methyl-propan-1-ol:

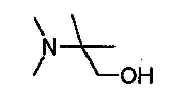

A mixture of 2-amino-2-methyl-1-propanol (5.00 g), formaldehyde solution, 36.5% in water (10.73 ml) and formic acid (11.00 ml) in water (20 ml) was stirred at 0 °C for 1 h and then heated to 90 °C for 4 h. The reaction mixture was concentrated *in vacuo*, the residue diluted with water (20 ml) and made alkaline (pH 14) by addition of 1 N NaOH. Furthermore, solid NaCl was added until saturation. The aqueous solution was extracted with dichloromethane (3 x 50 ml) and the combined organic layer was dried (Na₂SO₄) and concentrated *in vacuo*. Distillation under reduced pressure (40 mbar) afforded the desired compound as colorless oil.

### (S)-1-Methyl-piperidin-3-ol:

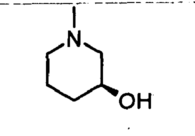

A mixture of (S)-3-hydroxypiperidine hydrochloride (4.00 g), formaldehyde solution, 36.5% in water (3.73 ml) and formic acid (2.19 ml) in water (20 ml) was kept under reflux overnight. The reaction mixture was concentrated *in vacuo*, the residue diluted with water (20 ml) and made alkaline (pH 14) by addition of 1 N NaOH. Furthermore, solid NaCl was added until saturation. The aqueous solution was extracted with dichloromethane (3 x 50 ml) and the combined organic layer was dried (Na₂SO₄) and concentrated *in vacuo*. Distillation under reduced pressure (10 mbar) afforded the desired compound as colorless liquid.

### Synthesis of B-C Moieties:

### B-C Moiety 1:

### Intermediate A1):

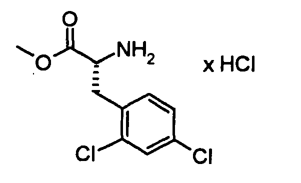

To a suspension of D-2,4-dichlorophenylalanine (10.00 g) in methanol (100 ml) was dropwise added thionylchloride (9.39 ml). During the course of the addition a clear solution was formed and the reaction started to reflux. The reaction mixture was kept under reflux for 2 h. After cooling to room temperature the mixture was evaporated to dryness at 40 °C. The crude product was triturated in diethyl ether, and the insoluble compound was filtered off, washed with diethyl ether, and finally dried *in vacuo* at room temperature over P₂O₅ overnight. The product was obtained in form of colorless needles.

### Intermediate B1):

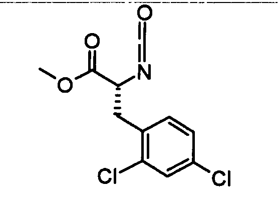

A 350 ml three-necked, flat-bottomed flask was equipped with a mechanical stirrer and charged with DCM (80 ml), saturated aqueous sodium bicarbonate solution (80 ml), and intermediate A1) (5.69 g). The biphasic mixture was cooled in an ice bath and stirred mechanically while triphosgene (1.96 g) was added in a single portion. The reaction mixture was stirred in the ice bath for 45 min and then poured into a 250 ml separatory funnel. The organic layer was collected, and the aqueous layer was extracted with three 20 ml portions of DCM. The combined organic layer was washed with water, dried over Na₂SO₄, filtered, and evaporated *in vacuo* to dryness to yield the crude product as a semisolid. The residue was purified by Kugelrohr distillation (200-240 °C, 0.04-0.08 mbar). The product was obtained as clear colorless oil.

### Intermediate C1):

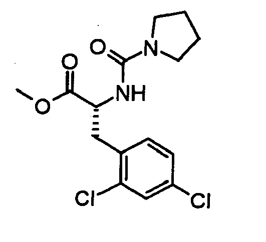

To an ice cooled solution of intermediate B1) (4.99 g) in DCM (50 ml) was added pyrrolidine (4.56 ml). After 10 minutes the ice bath was removed and stirring was continued for 4 h. The reaction mixture was evaporated *in vacuo*. The residue was redissolved in EtOAc and the organic layer was washed with 1 N HCl, water, sat. Na₂CO₃, water and brine. All the aqueous layers were extracted with EtOAc. The combined organic layer was dried over Na₂SO₄ and evaporated *in vacuo* to dryness.

### B-C Moiety 1:

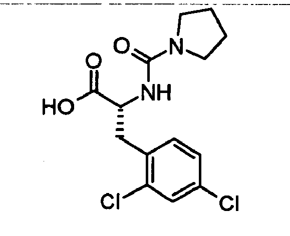

Intermediate C1) (6.28 g) was dissolved in MeOH (100 ml) and THF (30 ml) at 0 °C. A solution of lithium hydroxide monohydrate (1.53 g) in water (30 ml) was added dropwise over the course of 5 min. The mixture was stirred at 0 °C for 60 min and then acidified by adding 0.5 M HCl. The reaction mixture was extracted two times with EtOAc. The combined organic layer was washed two times with water and with brine, dried over Na₂SO₄ and evaporated *in vacuo*. The solid residue was triturated in Et₂O, then filtered off and washed with Et₂O. The product was obtained as a white solid.

### Synthesis of Example 2:

### Intermediate 2a):

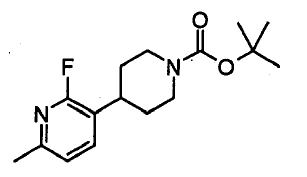

A flame dried Schlenk flask was charged with 3-bromo-2-fluoro-6-picoline (434 mg), [1,1'-bis(diphenylphosphino)ferrocene]dichloropalladium(II) complex with dichloromethane (56 mg), copper(I) iodide (26 mg), and dry *N,N*-dimethylacetamide (3 ml). The resulting mixture was degassed with alternating vacuum/argon purges. Then 1-tert-butoxycarbonylpiperidin-4-yl)(iodo)zinc (3.19 mmol, prepared as described above) was added. The mixture was degassed once again and then heated to 80 °C overnight. The mainpart of *N,N*-dimethylacetamide was then evaporated and the remainder was taken up in a mixture of EtOAc and water (50 ml each). The mixture was then filtered through Celite and transferred into a separatory funnel. The phases were separated and the aqueous layer was extracted with EtOAc (2 x 50 ml). The combined organic layer was washed with water and brine (100 ml each), dried (Na₂SO₄), filtered and concentrated *in vacuo*. The crude product was purified by column chromatography to furnish the desired compound in form of a colorless oil.

### Intermediate 2b):

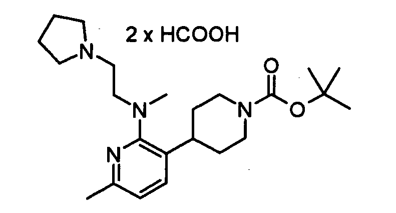

In a sealed tube were placed intermediate 2a) (0.55 g), methyl-(2-pyrrolidin-1-yl-ethyl)-amine (2.10 g), and N,N-diisopropylethylamine (286 µl). The reaction mixture was heated to 150 °C for 4 d. The mainpart of the volatiles was evaporated and the remainder taken up in EtOAc (100 ml). The solution was washed with NaHCO₃ (2 x 25 ml) and the combined water layer was re-extracted with EtOAc (25 ml). The organic layers were merged and washed with brine (25 ml), dried (Na₂SO₄), filtered and evaporated. The crude product was purified by column chromatography to isolate unreacted starting material and a mixed fraction containing the desired product. The desired compound was finally isolated by preparative HPLC under acidic conditions in form of a brownish resin.

### Intermediate 2c):

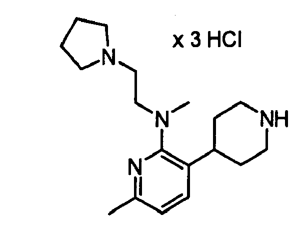

To Boc-protected intermediate 2b) (69 mg) in a mixture of DCM (1 ml) and MeOH (1 ml) was added hydrogen chloride, 4.0 M sol. in 1,4-dioxane (2 ml) and the solution was stirred at room temperature for 1 h. Evaporation of all volatiles and co-evaporation with toluene (2 x 5 ml), and acetone (5 ml) led to a beige solid, which was dried further in a desiccator over Sicapent overnight.

### Example 2:

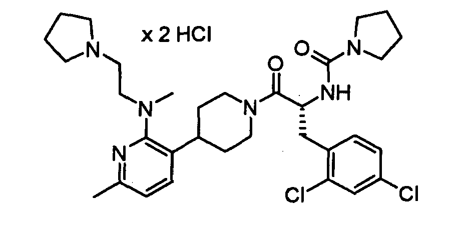

Intermediate 2c) (68 mg) and B-C Moiety 1 (60 mg), 1-hydroxybenzotriazole hydrate (32 mg) and N-methylmorpholine (58 µl) were dissolved in DMF (3 ml). After being stirred at room temperature for 30 min *N-*(3-dimethylaminopropyl)-*N*'-ethylcarbodiimide (48 mg) was added and stirring was continued for another hour. An additional amount of *N-*methylmorpholine (12 µl) was added and stirring was continued overnight. The reaction mixture was diluted with EtOAc (70 ml), washed with sat. Na₂CO₃ (3 x 25 ml), H₂O and brine (each with 25 ml). The organic layer was dried (Na₂SO₄) and the solvent removed in *vacuo.* Purification of the crude product by column chromatography furnished the corresponding amine in form of a yellowish resin. This was dissolved in EtOAc (1 ml) and treated with hydrogen chloride solution, 1.0 M in diethyl ether (114 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of an off-white solid.

### Synthesis of Example 4:

### Intermediate 4a):

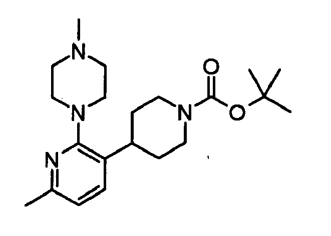

To a cooled solution (0 °C) of 1-methylpiperazine (257 µl) in dry THF (2 ml) was added n-butyllithium, 2.5 M in hexane (0.50 ml) dropwise via syringe. After being stirred at room temperature for 15 min, the resulting lithium amide was then added dropwise to a solution of 2a) (387 mg) in dry THF (2 ml) at 0 °C. After 2.5 h the reaction mixture was hydrolyzed with sat. NH₄Cl (2 ml) and diluted with EtOAc (70 ml). Extraction of the organic layer with NaHCO₃ (2 x 25 ml) and brine (25 ml), drying over Na₂SO₄, filtration and concentration in *vacuo* led to a yellow oil. This was purified by column chromatography to afford the desired compound in form of a yellowish resin.

### Intermediate 4b):

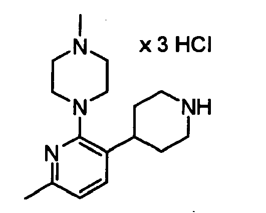

To Boc-protected intermediate 4a) (117 mg) in a mixture of DCM (1 ml) and MeOH (1 ml) was added hydrogen chloride, 4.0 M sol. in 1,4-dioxane (2 ml) and the solution was stirred at room temperature for 1 h. Evaporation of all volatiles and co-evaporation with toluene (2 x 5 ml), and acetone (5 ml) led to a beige solid, which was dried further in a desiccator over Sicapent overnight.

### Example 4:

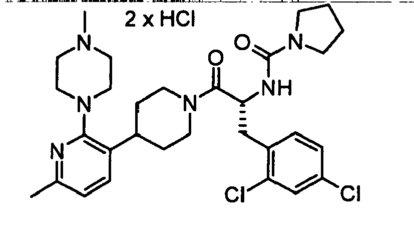

Intermediate 4b) (66 mg) and B-C Moiety 1 (70 mg), 1-hydroxybenzotriazole hydrate (37 mg) and *N*-methylmorpholine (68 µl) were dissolved in DMF (3 ml). After being stirred at room temperature for 30 min *N*-(3-dimethylaminopropyl)-*N*'-ethylcarbodiimide (56 mg) was added and stirring was continued for another hour. An additional amount of *N-*methylmorpholine (14 µl) was added and stirring was continued overnight. The reaction mixture was diluted with EtOAc (70 ml), washed with sat. Na₂CO₃ (3 x 25 ml), H₂O and brine (each with 25 ml). The organic layer was dried (Na₂SO₄) and the solvent removed in *vacuo*. Purification of the crude product by column chromatography furnished the corresponding amine in form of a yellowish solid. This was dissolved in EtOAc (1 ml) and treated with hydrogen chloride solution, 1.0 M in diethyl ether (214 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a white solid.

### Synthesis of Example 9:

### Intermediate 9a):

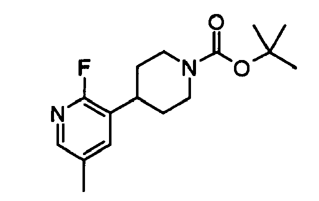

A flame dried Schlenk flask was charged with 3-bromo-2-fluoro-5-picoline (1.00 g), [1,1'-bis(diphenylphosphino)ferrocene]dichloropalladium(II) complex with dichloromethane (129 mg), copper(I) iodide (60 mg), and dry *N,N*-dimethylacetamide (7 ml). The resulting mixture was degassed with atternating vacuum/argon purges. Then 1-tert-butoxycarbonylpiperidin-4-yl)(iodo)zinc (7.37 mmol, prepared as described in above) was added. The mixture was degassed once again and then heated to 80 °C overnight. The mainpart of *N,N*-dimethylacetamide was then evaporated and the remainder was taken up in a mixture of EtOAc and water (100 ml each). The mixture was then filtered through Celite and transferred into a separatory funnel. The phases were separated and the water layer was extracted with EtOAc (3 x 50 ml). The combined organic layer was washed with water and brine (100 ml each), dried (Na₂SO₄), filtered and concentrated *in vacuo*. The crude product was purified by column chromatography to furnish the desired compound in form of a white solid.

### Intermediate 9b):

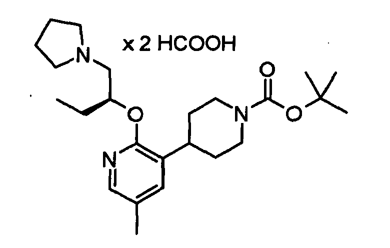

A solution of (S)-1-pyrrolidin-1-yl-butan-2-ol (191 mg) in DMF (1 ml) was added under argon at 0 °C to a suspension of sodium hydride, 60% dispersion in mineral oil (36 mg) in DMF (1 ml). The cooling bath was removed and the mixture stirred at room temperature for 1 h. Then a solution of intermediate 9a) (158 mg) in DMF (1 ml) was added and the reaction mixture was heated to 60 °C for 1 h. The reaction mixture was cooled to 0 °C and was then hyrolyzed with sat. NH₄Cl (1 ml). EtOAc (70 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo*. The crude product was purified by preparative HPLC under acidic conditions to afford the corresponding diformate in form of a yellowish oil.

### Intermediate 9c):

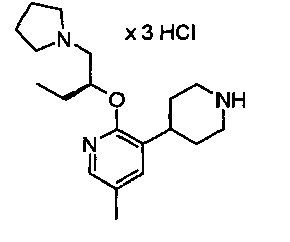

To Boc-protected intermediate 9b) (125 mg) in a mixture of DCM (2 ml) and MeOH (0.5 ml) was added hydrogen chloride, 4.0 M sol. in 1,4-dioxane (2 ml) and the solution was stirred at room temperature for 30 min. Evaporation of all volatiles and co-evaporation with toluene, acetone, and diethyl ether (5 ml each) led to a white solid, which was dried further in a desiccator over Sicapent overnight.

### Example 9:

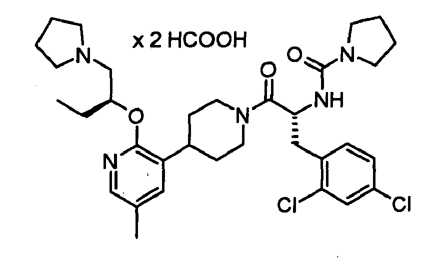

Intermediate 9c) (55 mg) and B-C Moiety 1 (55 mg), 1-hydroxybenzotriazole hydrate (29 mg) and *N*-methylmorpholine (54 µl) were dissolved in DMF (3 ml). After being stirred at room temperature for 30 min, *N*-(3-dimethylaminopropyl)-*N*'-ethylcarbodiimide (44 mg) was added and stirring was continued for another hour. An additional amount of *N-*methylmorpholine (11 µl) was added and stirring was continued overnight. The reaction mixture was diluted with EtOAc (70 ml), washed with sat. Na₂CO₃ (3 x 25 ml), water and brine (25 ml each). The organic layer was dried (Na₂SO₄) and the solvent removed *in vacuo*. The crude product was purified by preparative HPLC under acidic conditions to afford the corresponding diformate in form of a brownish resin.

### Synthesis of Example 10:

### Intermediate 10a):

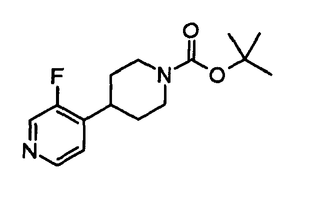

A flame dried Schlenk flask was charged with 3-fluoro-4-iodopyridine (1.00 g), [1,1'-bis(diphenylphosphino)ferrocene]dichloropalladium(II) complex with dichloromethane (110 mg), copper(I) iodide (51 mg), and dry *N,N*-dimethylacetamide (6 ml). The resulting mixture was degassed with alternating vacuum/argon purges. Then 1-tert-butoxycarbonylpiperidin-4-yl)(iodo)zinc (6.28 mmol, prepared as described above) was added. The mixture was degassed once again and then heated to 80 °C overnight. The mainpart of *N,N*-dimethylacetamide was then evaporated and the remainder was taken up in a mixture of EtOAc and water (each with 100 ml). The mixture was then filtered through Celite and transferred into a separatory funnel. The phases were separated and the water layer was extracted with EtOAc (3 x 30 ml). The combined organic layer was washed with water and brine (each with 75 ml), dried (Na₂SO₄), filtered and concentrated *in vacuo*. The crude product was purified by column chromatography to furnish the desired compound in form of a brownish solid.

### Intermediate 10b):

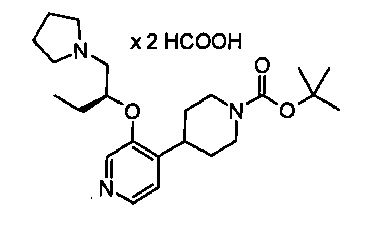

A solution of (S)-1-pyrrolidin-1-yl-butan-2-ol (156 mg) in DMF (2 ml) was added under argon at 0 °C to a suspension of sodium hydride, 60% dispersion in mineral oil (29 mg) in DMF (1 ml). The cooling bath was removed and the mixture stirred for 1 h at room temperature. Then a solution of intermediate 10a) (102 mg) in DMF (2 ml) was added and the reaction mixture was heated to 120 °C overnight. The reaction mixture was cooled to 0 °C and was then hyrolyzed with sat. NH₄Cl (1 ml). EtOAc (50 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo*. The crude product was purified by preparative HPLC under acidic conditions to afford the corresponding diformate in form of a yellowish resin.

### Intermediate 10c):

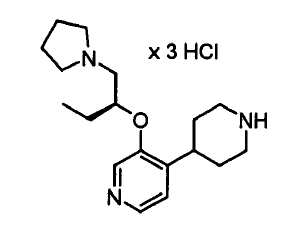

To Boc-protected intermediate 10b) (111 mg) in a mixture of DCM (1 ml) and MeOH (1 ml) was added hydrogen chloride, 4.0 M sol. in 1,4-dioxane (2 ml) and the solution was stirred at room temperature for 15 min. Evaporation of all volatiles and co-evaporation with toluene (2 x 5 ml), and acetone (5 ml) led to a beige solid, which was dried further in a desiccator over Sicapent overnight.

### Example 10:

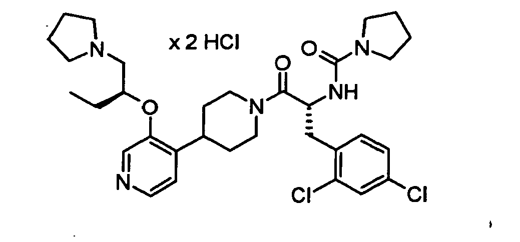

Intermediate 10c) (69 mg) and B-C Moiety 1 (58 mg), 1-hydroxybenzotriazole hydrate (31 mg) and *N*-methylmorpholine (57 µl) were dissolved in DMF (5 ml). After being stirred at room temperature for 30 min *N*-(3-dimethylaminopropyl)-*N*'-ethylcarbodiimide (47 mg) was added and stirring was continued for another hour. An additional amount of *N-*methylmorpholine (12 µl) was added and stirring was continued overnight. The reaction mixture was diluted with EtOAc (70 ml), washed with sat Na₂CO₃ (3 x 25 ml), H₂O and brine (each with 25 ml). The organic layer was dried (Na₂SO₄) and the solvent removed in *vacuo.* Purification of the crude product by column chromatography furnished the corresponding amine in form of a yellowish resin. This was dissolved in EtOAc (1 ml) and treated with hydrogen chloride solution, 1.0 M in diethyl ether (143 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a beige solid.

### Synthesis of Example 11:

### Intermediate 11a):

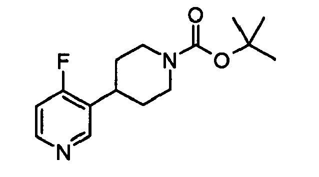

A flame dried Schlenk flask was charged with 4-fluoro-3-iodo-pyridine (1.00 g), [1,1'-bis(diphenylphosphino)ferrocene]dichloropalladium(II) complex with dichloromethane (110 mg), copper(I) iodide (51 mg), and dry *N,N*-dimethylacetamide (6 ml). The resulting mixture was degassed with alternating vacuum/argon purges. Then 1-tert-butoxycarbonylpiperidin-4-yl)(iodo)zinc (6.28 mmol, prepared as described above) was added. The mixture was degassed once again and then heated to 80 °C overnight. The mainpart of *N,N*-dimethylacetamide was then evaporated and the remainder was taken up in a mixture of EtOAc and water (each with 100 ml). The mixture was then filtered through Celite and transferred into a separatory funnel. The phases were separated and the water layer was extracted with EtOAc (3 x 30 ml). The combined organic layer was washed with water and brine (each with 75 ml), dried (Na₂SO₄), filtered and concentrated *in vacuo*. The crude product was purified by column chromatography to furnish the desired compound in form of a brownish solid.

### Intermediate 11b):

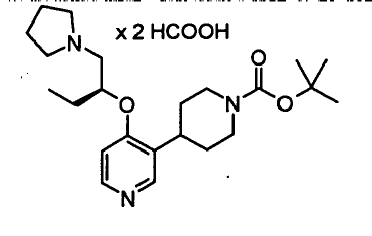

A solution of (S)-1-pyrrolidin-1-yl-butan-2-ol (187 mg) in DMF (2 ml) was added under argon at 0 °C to a suspension of sodium hydride, 60% dispersion in mineral oil (35 mg) in DMF (1 ml). The cooling bath was removed and the mixture stirred for 1 h at room temperature. Then a solution of intermediate 11a) (122 mg) in DMF (2 ml) was added and the reaction mixture was heated to 60°C for 1 h. The reaction mixture was cooled to 0 °C and was then hyrolyzed with sat. NH₄Cl (1 ml). EtOAc (70 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo.* The crude product was purified by preparative HPLC under acidic conditions to afford the corresponding diformate in form of a yellowish resin.

### Intermediate 11c):

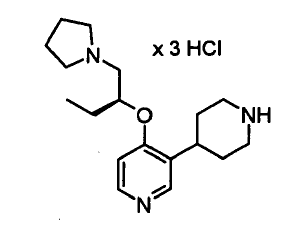

To Boc-protected intermediate 11b) (151 mg) in a mixture of DCM (1 ml) and MeOH (1 ml) was added hydrogen chloride, 4.0 M sol. in 1,4-dioxane (2 ml) and the solution was stirred at room temperature for 2 h. Evaporation of all volatiles and co-evaporation with toluene (2 x 5 ml), acetone, and ether (5 ml each) led to a beige solid, which was dried further in a desiccator over Sicapent overnight.

### Example 11:

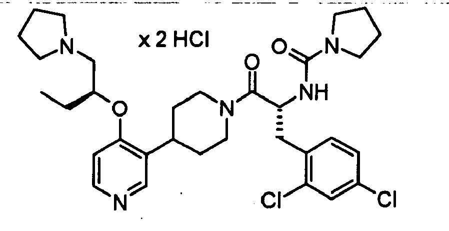

Intermediate 11c) (68 mg) and B-C Moiety 1 (61 mg), 1-hydroxybenzotriazole hydrate (33 mg) and *N*-methylmorpholine (59 µl) were dissolved in DMF (3 ml). After being stirred at room temperature for 30 min *N*-(3-dimethylaminopropyl)-*N*'-ethylcarbodiimide (49 mg) was added and stirring was continued for another hour. An additional amount of N-methylmorpholine (12 µl) was added and stirring was continued overnight. The reaction mixture was diluted with EtOAc (70 ml), washed with sat. Na₂CO₃ (3 x 25 ml), water and brine (each with 25 ml). The organic layer was dried (Na₂SO₄) and the solvent removed *in vacuo.* Purification of the crude product by column chromatography furnished the corresponding amine in form of a brownish resin. This was dissolved in EtOAc (1 ml) and treated with hydrogen chloride solution, 1.0 M in diethyl ether (178 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of an off-white solid.

### Synthesis of Example 12:

### Intermediate 12a):

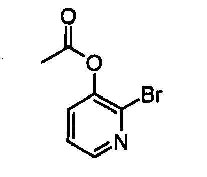

A solution of 2-bromo-3-pyridinol (1.04 g) in pyridine (2.00 ml) and dry DCM (20 ml) was cooled to 0 °C. Acetic anhydride (1.76 ml) was added and the reaction mixture was stirred at room temperature overnight. All volatiles were evaporated and the remainder was suspended in water (30 ml). After being stirred at room temperature for 1 h, the suspension was diluted with 1 N NaHCO₃ (50 ml) and extracted with EtOAc (3 x 50 ml). The combined organic layer was washed with 1 N NaHCO₃, and brine (50 ml each), dried (MgSO₄), filtered and evaporated to afford the desired compound in form of an amber oil.

### Intermediate 12b):

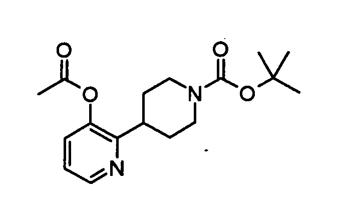

A flame dried Schlenk flask was charged with intermediate 12a) (2.00 g), [1,1'-bis(diphenylphosphino)ferrocene]dichloropalladium(II) complex with dichloromethane (227 mg), copper(I) iodide (106 mg), and dry *N,N*-dimethylacetamide (10 ml). The resulting mixture was degassed with alternating vacuum/argon purges. Then 1-tert-butoxycarbonylpiperidin-4-yl)(iodo)zinc (13.0 mmol, prepared as described above) was added. The mixture was degassed once again and then heated to 80 °C overnight. The mainpart of *N,N*-dimethylacetamide was then evaporated and the remainder was taken up in a mixture of EtOAc and water (each with 200 ml). The mixture was then filtered through Celite and transferred into a separatory funnel. The phases were separated and the water layer was extracted with EtOAc (3 x 75 ml). The combined organic layer was washed with water and brine (150 ml each), dried (Na₂SO₄), filtered and concentrated *in vacuo.* The crude product was purified by column chromatography to furnish the desired compound in form of a brownish solid.

### Intermediate 12c):

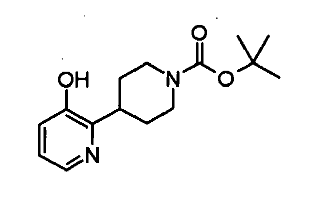

A solution of intermediate 12b) (1.97 g) in MeOH (60 ml) was cooled to 0 °C. A solution of lithium hydroxide (368 mg) in water (30 ml) was added and the reaction mixture was stirred at 0 °C for 10 min. The pH was adjusted to ∼7 by dropwise addition of 0.5 M HCl at 0 °C. Water (100 ml) was added and an extraction with EtOAc (3 x 100 ml) was carried out. After washing with brine (150 ml), drying (Na₂SO₄), filtration and evaporation the desired compound was obtained as brownish foam.

### Intermediate 12d):

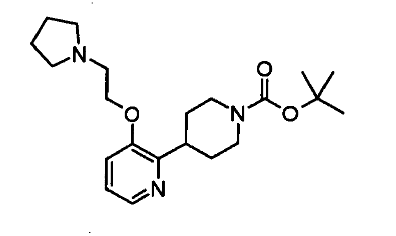

A solution of intermediate 12c) (207 mg), 1-(2-chloroethyl)pyrrolidine hydrochloride (190 mg), and cesium carbonate (969 mg) in DMF (10 ml) was stirred at room temperature overnight. All volatiles were evaporated and the residue was partitioned between EtOAc and sat. NaHCO₃ (50 ml each). The aqueous layer was extracted with EtOAc (2 x 25 ml). The combined organic layer was washed with water and brine (25 ml each), dried (Na₂SO₄) and evaporated to afford the desired compound as brownish resin.

### Intermediate 12e):

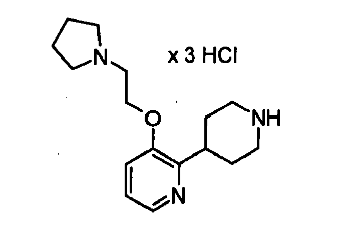

To Boc-protected intermediate 12d) (253 mg) in a mixture of DCM (2 ml) and MeOH (0.5 ml) was added hydrogen chloride, 4.0 M sol. in 1,4-dioxane (2 ml) and the solution was stirred at room temperature for 15 min. Evaporation of all volatiles and co-evaporation with toluene, acetone, and ether (5 ml each) led to a beige solid, which was dried further in a desiccator over Sicapent overnight.

### Example 12:

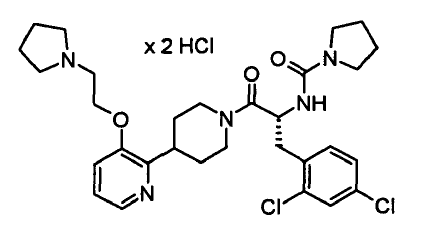

Intermediate 12e) (180 mg) and B-C Moiety 1 (201 mg), 1-hydroxybenzotriazole hydrate (107 mg) and N-methylmorpholine (195 µl) were dissolved in DMF (5 ml). After being stirred at room temperature for 30 min *N*-(3-dimethylaminopropyl)-*N*'-ethylcarbodiimide (161 mg) was added and stirring was continued for another hour. An additional amount of *N*-methylmorpholine (41 µl) was added and stirring was continued overnight. The reaction mixture was diluted with EtOAc (100 ml), washed with sat. Na₂CO₃ (3 x 50 ml), water and brine (each with 50 ml). The organic layer was dried (Na₂SO₄) and the solvent removed *in vacuo.* Purification of the crude product by column chromatography furnished the corresponding amine in form of a brownish resin. This was dissolved in EtOAc (1 ml) and treated with hydrogen chloride solution, 1.0 M in diethyl ether (564 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a white solid.

### Synthesis of Example 19:

### Intermediate 19a):

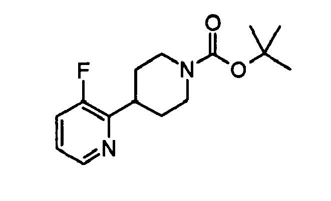

A flame dried Schlenk flask was charged with 2-bromo-3-fluoropyridine (500 mg), [1,1'-bis(diphenylphosphino)ferrocene]dichloropalladium(II) complex with dichloromethane (70 mg), copper(I) iodide (32 mg), and dry *N,N*-dimethylacetamide (4 ml). The resulting mixture was degassed with alternating vacuum/argon purges. Then 1-tert-butoxycarbonylpiperidin-4-yl)(iodo)zinc (3.99 mmol, prepared as described above) was added. The mixture was degassed once again and then heated to 80 °C overnight. The mainpart of *N,N*-dimethylacetamide was then evaporated and the remainder was taken up in a mixture of EtOAc and water (each with 75 ml). The mixture was then filtered through Celite and transferred into a separatory funnel. The phases were separated and the water layer was extracted with EtOAc (3 x 25 ml). The combined organic layer was washed with water and brine (75 ml each), dried (Na₂SO₄), filtered and concentrated *in vacuo.* The crude product was purified by column chromatography to furnish the desired compound in form of a brownish oil.

### Intermediate 19b):

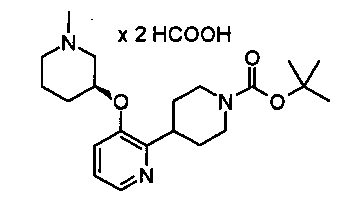

A solution of (S)-1-methyl-piperidin-3-ol (228 mg) in DMF (2 ml) was added under argon at 0 °C to a suspension of sodium hydride, 60% dispersion in mineral oil (53 mg) in DMF (1 ml). The cooling bath was removed and the mixture stirred at room temperature for 1 h. Then a solution of intermediate 19a) (185 mg) in DMF (2 ml) was added and the reaction mixture was heated to 120 °C overnight. The reaction mixture was cooled to 0 °C and then hyrolyzed with sat. NH₄Cl (1 ml). EtOAc (50 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo.* The crude product was purified by preparative HPLC under acidic conditions to afford the corresponding diformate in form of a yellowish resin.

### Intermediate 19c):

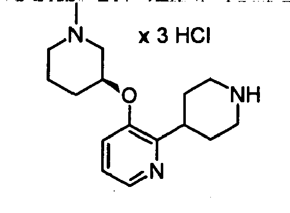

To Boc-protected intermediate 19b) (148 mg) in a mixture of DCM (1 ml) and MeOH (1 ml) was added hydrogen chloride, 4.0 M sol. in 1,4-dioxane (2 ml) and the solution was stirred at room temperature for 15 min. Evaporation of all volatiles and co-evaporation with toluene (2 x 5 ml), and acetone (5 ml) led to a beige solid, which was dried further in a desiccator over Sicapent overnight.

### Example 19:

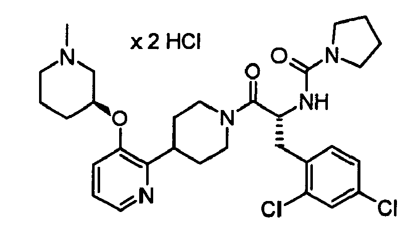

Intermediate 19c) (93 mg) and B-C Moiety 1 (83 mg), 1-hydroxybenzotriazole hydrate (44 mg) and *N*-methylmorpholine (81 µl) were dissolved in DMF (5 ml). After being stirred at room temperature for 30 min *N*-(3-dimethylaminopropyl)-*N*'-ethylcarbodiimide (67 mg) was added and stirring was continued for another hour. An additional amount of N-methylmorpholine (17 µl) was added and stirring was continued overnight. The reaction mixture was diluted with EtOAc (70 ml), washed with sat. Na₂CO₃ (3 x 25 ml), water and brine (each with 25 ml). The organic layer was dried (Na₂SO₄) and the solvent removed *in vacuo.* Purification of the crude product by column chromatography furnished the corresponding amine in form of a yellowish resin. This was dissolved in EtOAc (1 ml) and treated with hydrogen chloride solution, 1.0 M in diethyl ether (238 µl). The resulting suspension was diluted with hexane (5 ml) in order to obtain a complete precipitation of the corresponding dihydrochloride. The solid was filtered off, washed with hexane, and dried in a desiccator over Sicapent overnight to provide the desired compound in form of a white solid.

### Synthesis of Example 20:

### Intermediate 20a):

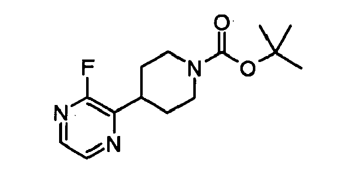

A flame dried Schlenk flask was charged with 2-fluoro-3-iodo-pyrazine (1.00 g), [1,1'-bis(diphenylphosphino)ferrocene]dichloropalladium(II) complex with dichloromethane (109 mg), copper(I) iodide (51 mg), and dry *N,N*-dimethylacetamide (6 ml). The resulting mixture was degassed with alternating vacuum/argon purges. Then 1-tert-butoxycarbonylpiperidin-4-yl)(iodo)zinc (6.25 mmol, prepared as described above) was added. The mixture was degassed once again and then heated to 80 °C overnight. The mainpart of *N,N*-dimethylacetamide was then evaporated and the remainder was taken up in a mixture of EtOAc and water (100 ml each). The mixture was then filtered through Celite and transferred into a separatory funnel. The phases were separated and the water layer was extracted with EtOAc (3 x 30 ml). The combined organic layer was washed with water and brine (75 ml each), dried (Na₂SO₄), filtered and concentrated *in vacuo.* The crude product was purified by column chromatography to furnish the desired compound in form of a brownish oil.

### Intermediate 20b):

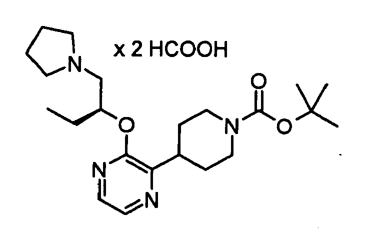

A solution of (S)-1-pyrrolidin-1-yl-butan-2-ol (211 mg) in DMF (2 ml) was added under argon at 0 °C to a suspension of sodium hydride, 60 % dispersion in mineral oil (39 mg) in DMF (1 ml). The cooling bath was removed and the mixture stirred for 1 h at room temperature. Then a solution of intermediate 20a) (138 mg) in DMF (2 ml) was added and the reaction mixture was heated to 60 °C for 1 h. The reaction mixture was cooled to 0 °C and was then hyrolyzed with sat. NH₄Cl (1 ml). EtOAc (70 ml) was added and the mixture was washed with NaHCO₃ (2 x 25 ml). The combined aqueous layer was re-extracted with EtOAc (25 ml) and the combined organic layer then washed with brine (25 ml), dried (Na₂SO₄), filtered and concentrated *in vacuo.* The crude product was purified by preparative HPLC under acidic conditions to afford the corresponding formate in form of a yellowish resin.

### Intermediate 20c):

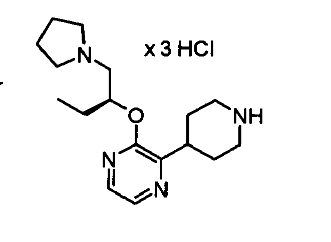

To Boc-protected intermediate 20b) (181 mg) in a mixture of DCM (1 ml) and MeOH (1 ml) was added hydrogen chloride, 4.0 M sol. in 1,4-dioxane (2 ml) and the solution was stirred at room temperature for 2 h. Evaporation of all volatiles and co-evaporation with toluene (2 x 5 ml), acetone (5 ml), and ether (5 ml) led to a beige solid, which was dried further in a desiccator over Sicapent overnight.

### Example 20:

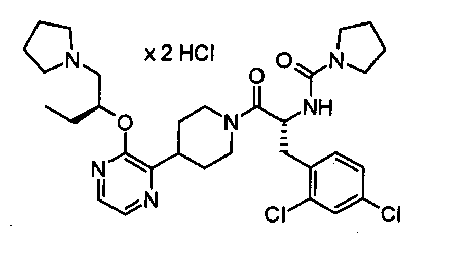

Intermediate 20c) (67 mg) and B-C Moiety 1) (68 mg), 1-hydroxybenzotriazole hydrate (36 mg) and *N-*methylmorpholine (66 µl) were dissolved in DMF (3 ml). After being stirred for 30 min at room temperature *N*-(3-dimethylaminopropyl)-*N*'-ethylcarbodiimide (54 mg) was added and stirring was continued for another hour. An additional amount of N-methylmorpholine (14 µl) was added and stirring was continued overnight. The reaction mixture was diluted with EtOAc (70 ml), washed with sat. Na₂CO₃ (3 x 25 ml), water and brine (each with 25 ml). The organic layer was dried (Na₂SO₄) and the solvent removed *in vacuo.* Purification of the crude product by column chromatography furnished the corresponding amine in form of a brownish resin. This was dissolved in EtOAc (1 ml) and treated with hydrogen chloride solution, 1.0 M in diethyl ether (178 µl). The resulting suspension was diluted with hexane (5 ml) in order to obtain a complete precipitation of the corresponding hydrochloride. The solid was filtered off, washed with hexane, and dried in a desiccator over Sicapent overnight to provide the desired compound in form of an off-white solid.

### Biological Assays

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4°C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have IC₅₀ values less than 2 µM.

**Table 6: Biological data for selected examples of the invention**

| Example | hMC-4R binding assay IC₅₀/nM | hMC-4R functional assay EC₅₀/nM | % activation functional assay |
|---|---|---|---|
| SHU-9119 | 1.9 | | 7 |
| NDP-α-MSH | 1.1 | 3.4 | 100 |
| 1 | 14 | - | 0 |
| 3 | 300 | - | 0 |
| 4 | 8.2 | - | 0 |
| 5 | 39 | - | 0 |
| 10 | 23 | - | 0 |
| 11 | 90 | - | 0 |
| 12 | 130 | | - 0 |
| 19 | 19 | 270 | -32 |
| 20 | 56 | - | 0 |

### C. In Vivo Food Intake Models

### 1. Spontaneous Feeding Paradigm

Food intake in rats is measured after i.p., s.c. or p.o. administration of the test compound (see e.g. A.S. Chen et al. Transgenic Res 2000 Apr ;9(2):145-154).

### 2. Model of LPS-Induced Anorexia and Tumor-Induced Cachexia

Prevention or amelioration of anorexia induced by either lipopolysaccharide (LPS) administration or cachexia induced by tumor growth is determined upon i.p. or p.o. administration of test compounds to rats (see e.g. D.L. Marks, N. Ling, and R.D. Cone, Cancer Res 2001 Feb 15;61 (4):1432-1438).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how, and if, an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations to drug treated evaluations for individual animals. Groups of a minimum of 4 animals are utilized to reduce variability. Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in K.E. McKenna et al, A Model For The Study of Sexual Function In Anesthetized Male And Female Rats, Am. J. Physiol. (Regulatory Integrative Comp. Physiol 30): R1276-R1285, 1991; K.E. McKenna et al, Modulation By Peripheral Serotonin of The Threshold For Sexual Reflexes In Female Rats, Pharm. Bioch. Behav., 40:151-156, 1991; and L.K. Takahashi et al, Dual Estradiol Action In The Diencephalon And The Regulation of Sociosexual Behavior In Female Golden Hamsters, Brain Res., 359: 194-207, 1985.

### Examples of a Pharmaceutical Composition

As a specific embodiment of an oral composition of a compound of the present invention, 23 mg of Example 6 is formulated with sufficient finely divided lactose to provide a total amount of 580 to 590 mg to fill a size 0 hard gelatin capsule.

As another specific embodiment of an oral composition of a compound of the present invention, 28 mg of Example 12 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound according to formula (I) 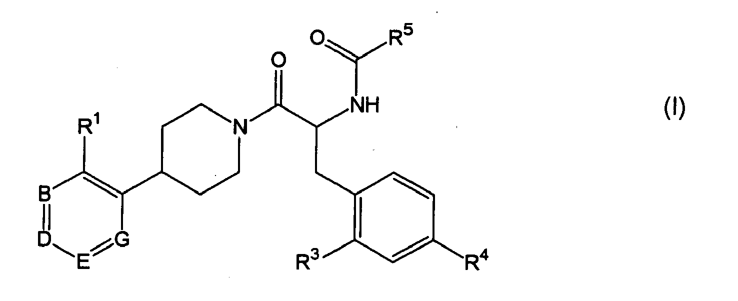 and the enantiomers, diastereomers, tautomers, solvates and pharmaceutically acceptable salts thereof,
wherein
R¹ is -N(R¹⁰)-(C(R⁶)₂)ₘ-T
-(C(R⁶)₂)ₗ-T, or
-O-(C(R⁶)₂)ₘ-T;
R⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T is NR⁷R⁸,
morpholine, 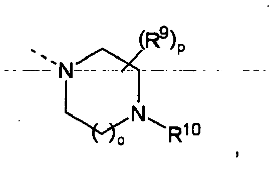 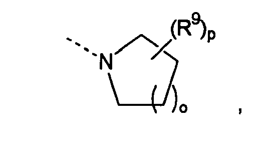 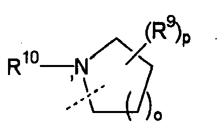 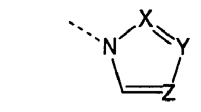 or 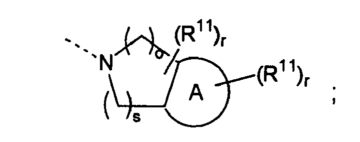
R⁷ and R⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁹ is independently selected from
halogen,
CN,
OH,
C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH;
R¹⁰ is H, or
C₁-C₆-alkyl;
R¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X is CH or N;
Y is CH or N;
Z is CH or N;
A is a 3-7-membered saturated, unsaturated or aromatic ring containing 0-2 nitrogen atoms;
B is CR² or N;
G is CR² or N;
D is CR² or N;
E is CR² or N;
with the proviso that one or two of the variables B, G, D and E must be N;
R² is independently selected from
H,
F,
Cl,
CH₃,
OCH₃, and
CF₃;
R³ is H,
Cl,
F, or
CH₃;
R⁴ is Cl or F;
R⁵ is 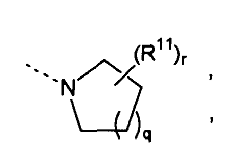 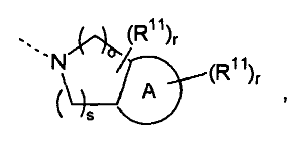 morpholine, optionally substituted by 1 to 3, same or different substituents R¹⁴, or
NR¹²R¹³;
R¹² and R¹³ are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
R¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂;
I is 0, 1, 2, 3, or 4;
m is 0, 1, 2, 3, or 4;
o is 0, 1, or 2;
p is 0, 1, 2, 3, or 4;
q is 0, 1, 2, or 3;
r is 0, 1, 2, 3, or 4 and
s is 1, or 2.

2. The compound of claim 1 according to formula (I') 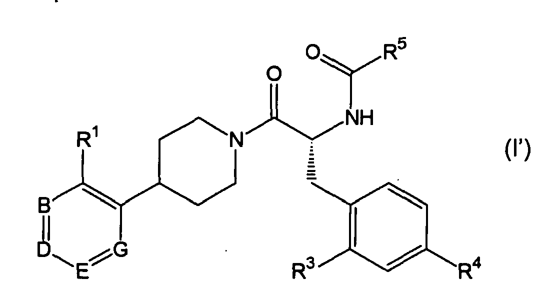 wherein B, G, D, E, R¹, R³, R⁴ and R⁵ are as defined in claim 1.

3. The compound of claim 1 or 2, wherein at least one of R⁷ and R⁸ is selected from
C₂₋₆-alkenyl,
C₂₋₆-alkinyl, and
C₂₋₆-alkylene-O-C₁₋₆-alkyl.

4. The compound of any of claims 1 to 3, wherein
R² is selected from H, F, Cl and CH₃.

5. The compound of any of claims 1 to 4 wherein
I is 2 or 3, or
m is 2 or 3.

6. The compound of any of claims 1 to 5 as medicament.

7. Use of the compound of any of claims 1 to 5 for the preparation of a medicament for the treatment or prophylaxis of disorders, diseases or conditions responsive to the inactivation or activation of the melanocortin-4 receptor in a mammal.

8. Use according to claim 7 for the preparation of a medicament for the treatment or prophylaxis of cancer cachexia.

9. Use according to claim 7 for the preparation of a medicament for the treatment or prophylaxis of muscle wasting.

10. Use according to claim 7 for the preparation of a medicament for the treatment or prophylaxis of anorexia.

11. Use according to claim 7 for the preparation of a medicament for the treatment or prophylaxis of anxiety and/or depression.

12. Use according to claim 7 for the preparation of a medicament for the treatment or prophylaxis of obesity.

13. Use according to claim 7 for the preparation of a medicament for the treatment or prophylaxis of diabetes mellitus.

14. Use according to claim 7 for the preparation of a medicament for the treatment or prophylaxis of male or female sexual dysfunction.

15. Use according to claim 7 for the preparation of a medicament for the treatment or prophylaxis of erectile dysfunction.

16. A pharmaceutical composition comprising a compound of any of claims 1 to 5 and a pharmaceutically acceptable carrier.
